(19) Europäisches Patentamt
European Patent Office
Office européen des brevets

(11) Publication number: 0 500 934 A1

# (12) EUROPEAN PATENT APPLICATION published in accordance with Art. 158(3) EPC

(21) Application number: **90916075.6**

(51) Int. Cl.[5]: **C07D 263/58**, A01N 43/76

(22) Date of filing: **31.10.90**

(86) International application number: **PCT/JP90/01403**

(87) International publication number: **WO 91/06544 (16.05.91 91/11)**

(30) Priority: **31.10.89 JP 281978/89**

(43) Date of publication of application: **02.09.92 Bulletin 92/36**

(84) Designated Contracting States: **IT**

(71) Applicant: **HOKKO CHEMICAL INDUSTRY CO. LTD.**
**4-20, Nihonbashi Hongokucho 4-chome**
**Chuo-ku Tokyo 103(JP)**

(72) Inventor: **OHYAMA, Hiroshi**
**B-22-19, 36-20, Tsutsumi**
**Chigasaki-shi, Kanagawa 253(JP)**
Inventor: **MORITA, Ken**
**Takamura Danchi 16-708, 203, Takamura**
**Hiratsuka-shi, Kanagawa 254(JP)**
Inventor: **NAKAMURA, Toshiki**
**Hokko Kagaku-ryo, 2385, Toda**
**Atsugi-shi, Kanagawa 243(JP)**
Inventor: **NIITSUMA, Shiro**
**Villa Heights T-1-102, 2171, Sakai**
**Atsugi-shi, Kanagawa 243(JP)**
Inventor: **SHIMOZONO, Takuro**
**Hokko Kagaku-ryo, 2385, Toda**
**Atsugi-shi, Kanagawa 243(JP)**
Inventor: **YOSHIZAWA, Hirokazu**
**Hokko Kagaku-ryo, 2385, Toda**
**Atsugi-shi, Kanagawa 243(JP)**
Inventor: **YAMAMURA, Hiroshi**
**3-5, Ishigamidai 2-chome, Ohiso-machi**
**Naka-gun, Kanagawa 259-01(JP)**

(74) Representative: **Corradini, Corrado**
**4, Via Dante Alighieri**
**I-42100 Reggio Emilia(IT)**

## (54) ARALKYLOXYAMINE DERIVATIVE AND HERBICIDE.

(57) A new aralkyloxyamine derivative of general formula (I), which has an excellent herbicidal activity and is useful as a herbicide for controlling weeds growing in both paddy and upland fields, wherein A represents alkylene and R represents hydrogen, lower alkyl, lower alkenyl, lower alkynyl, cycloalkyl, lower alkoxy-lower alkyl, lower alkylthio-lower alkyl, lower alkoxycarbonyl-lower alkyl, cyano-lower alkyl, lower alkylcarbonyl-lower alkyl, or phenyl-lower alkyl.

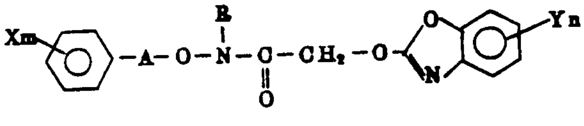

(I)

EP 0 500 934 A1

## TECHNICAL FIELD

This invention relates to novel aralkyloxyamine derivatives and also to a herbicidal composition containing said derivative or derivatives as active ingredient. More specifically, this invention is concerned with novel aralkyloxyamine derivatives represented by the general formula (I) shown hereinafter, as well as a herbicidal composition containing said derivative(s) as active ingredient. This invention is therefore useful in the fields of chemical industry and agriculture, especially in the field of industry for production of agricultural chemicals.

## BACKGROUND ART

Some benzoxazole derivatives having the oxyamide structure are described in some publicaitons which have been published up to date. For example, the compounds represented by the following general formula (A) are disclosed in Japanese Patent Application first publicaiton "Kokai" No. Sho-56-86179 and are described to be effective as a herbicide:

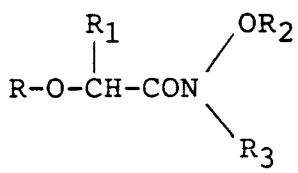

(A)

In the above formula (A), R represents a 5-membered heterocyclic group, including a fused heterocyclic group. Benzoxazole group is mentioned as one of such heterocyclic group. There are also disclosed such compounds of the formula (A) where $R_1$ is a hydrogen atom, $R_2$ is a lower alkyl group, a lower alkenyl group or a lower alkoxyalkyl group, and $R_3$ is a lower alkyl group, a lower alkenyl gorup, a cycloalkyl group or a lower alkoxyalkyl group.

Further, in Japanese Patent Application first publication "Kokai" No. Sho-57-4903, the compounds shown by the following general formula (B) are described there to be useful as plant growth regulators.

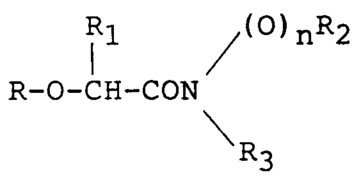

(B)

In the above formula (B), R stands for a 5-membered heterocyclic group, including a fused heterocyclic group. Benzoxazole group is mentioned as one of such heterocyclic group. There are also disclosed such compounds of the formula (B) where $R_1$ is a hydrogen atom, $R_2$ is a lower alkyl group, a lower alkenyl group or a lower alkoxyalkyl group, and $R_3$ is a lower alkyl group, a lower alkenyl group or a cycloalkyl group.

However, the aralkyloxyamine derivatives of the general formula (I) according to the present invention have not been disclosed in any publications and are hence novel compounds.

Herbicidal compounds which have conventionally been used in greatest amounts as herbicides are alachlor and butachlor, which both are haloacetonitride derivatives (see the specifications of U.S. Patent Nos. 3,442,945 and 3,457,620). Alachlor is a common name of 2-chloro-2',6'-diethyl-N-(methoxymethyl)-acetanilide having the following formula:

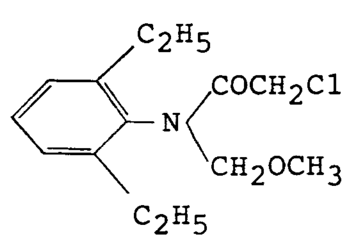

Buthachlor is a common name of 2-chloro-2',6'-diethyl-N-(butoxymethyl)acetanilide having the following formula:

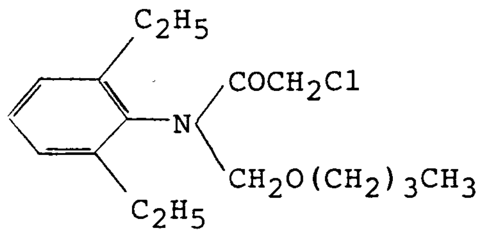

These herbicidal compounds have broad herbicidal spectra and strongly act on graminaceous weeds in particular. Alachlor is used widely as a herbicide for use in fields of upland crops such as soybean and corn, while butachlor is used mainly as a herbicide for use in paddy fields of rice plants. Although these herbicidal compounds have high herbicidal activities, they can cause phytotoxicity or injury on crop plants in many instances. There is thus a long standing-desire of users for development of herbicidal compounds which would have high herbicidal activities but are yet free from the danger of phytotoxicity. In addition, alachlor and butachlor are used worldwide in extremely large amounts, so that their influence to the environment, for example, the contamination of underground water and river waters now give increasing problems. It is therefore not preferred that such one and some particular herbicidal compound is applied in large amounts over many years. There occurs accordingly a strong demand for development of novel herbicidal compounds which are different in their chemical structure from alachlor and butachlor.

However, the known compounds having the formula (A) and formula (B) shown above, of which a part has a chemical structure similar to that of the compound of the formula (I) according to the present invention, have some drawbacks that said known compounds are not preferable as herbicides in practice, in account of their own herbicidal effects and phytotoxicity, as will be demonstrated in Test Examples described hereinafter.

## DISCLOSURE OF THE INVENTION

Accordingly, an object of the present invention is to provide novel herbicidal compounds which are useful as a herbicide for use in paddy fields of rice plant and also as a herbicide for use in fields of upland crops and which further have more excellent herbicidal activities and safety, as compared to the known conventional herbicidal compounds of the haloanilide type and are different in their chemical structure from the known herbicidal compounds commercially available up to the date.

In an attempt to achieve the above-described object, we, the present inventors, have synthesized numerous aralkyloxyamine derivatives and conducted extensive investigations on their utility as the herbicides. As a result, we have now found that the aralkyloxyamine derivatives represented by the general formula (I) shown hereinafter are novel compounds which are not disclosed in any publications but have higher herbicidal activities and higher safety to crops and useful living organisms, as compared with the known compounds of the formula (A) and formula (B) having a similar chemical structure, and which can achieve the object of this invention described above.

In the first aspect of this invention, therefore, there is provided an aralkyloxyamine derivative represented by the following general formula:

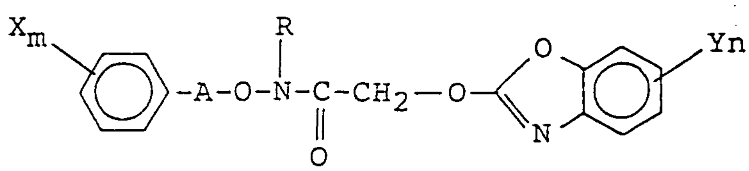

(I)

wherein X means a hydrogen atom, a halogen atom, a lower alkyl group, a lower alkoxy group, a lower haloalkyl group, a lower haloalkoxy group, a lower alkylthio group, a lower alkylsulfinyl group, a lower alkylsulfonyl group, a lower alkylcarbonyl group, a nitro group or cyano group or a lower alkoxycarbonyl group, or X means a phenoxy group which may be substituted by a halogen atom, a lower alkyl group, a

lower alkoxy group or a lower haloalkyl group, m stands for 1, 2 or 3, and when a plurality of the group X are present, these groups X may be the same or different from each other, and Y denotes a hydrogen atom, a halogen atom, a lower alkyl group, a lower alkoxy group, a lower haloalkyl group, a lower haloalkoxy group, a lower alkylthio group, a lower alkylcarbonyl group, a nitro group or cyano group, n stands for 1 or 2, and when a plurality of the group Y are present, these groups Y may be the same or different from each other, and A means an alkylene group, and R represents a hydrogen atom, a lower alkyl group, a lower alkenyl group, a lower alkynyl group, a cycloalkyl group, a lower alkoxy-lower alkyl group, a lower alkylthio-lower alkyl group, a lower alkoxycarbonyl-lower alkyl group, a cyano-lower alkyl group, a lower alkylcarbonyl-lower alkyl group or a phenyl-lower alkyl group.

The lower alkyl group, lower alkenyl group and lower alkynyl group as referred to herein may each be either linear or branched. The term "lower" as used herein means those groups having 1 to 6 carbon atoms. Further, the cycloalkyl group may contain 3 to 6 carbon atoms. Preferred examples of the phenyl-lower alkyl group include benzyl group and phenethyl group.

The alkylene group which is represented by A may preferably be an alkylene group containing 1 to 10 carbon atoms, especially a lower alkylene group containing 1 to 6 carbon atoms. The alkylene group may bear thereon a lower alkyl group or a lower alkenyl group as the side-chain.

In the second aspect of this invention, there is further provided a herbicidal composition comprising the aralkyloxyamine derivative of the above-mentioned general formula (I) as an active ingredient, and a carrier as mixed with the active ingredient.

## BEST MODE OF WORKING THE INVENTION

Representative particular examples of the compound of the general formula (I) according to this invention are now described in Table 1 below.

Compound Nos. indicated in Table 1 will be referred to again in Examples and Test Examples which are described hereinafter.

Table 1

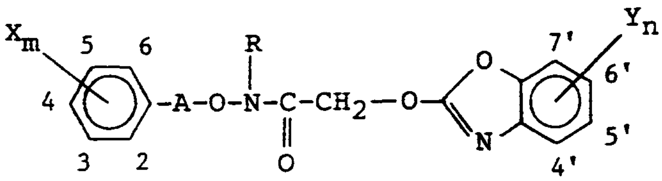

(I)

| Compound No. | $X_m$ | $Y_n$ | A | R | Physical Data (melting point or refractive index) |
|---|---|---|---|---|---|
| 1 | H— | H— | $-CH_2-$ | H— | mp 95-97.5°C |
| 2 | H— | H— | $-CH_2-$ | $CH_3-$ | mp 79-81°C |
| 3 | H— | H— | $-CH_2-$ | $C_2H_5-$ | mp 60-62°C |
| 4 | H— | H— | $-CH_2-$ | n-$C_3H_7-$ | $n_D^{23}$ 1.5704 |
| 5 | H— | H— | $-CH_2-$ | i-$C_3H_7-$ | mp 55-56°C |
| 6 | H— | H— | $-CH_2-$ | n-$C_4H_9-$ | mp 72-73°C |
| 7 | H— | H— | $-CH_2-$ | i-$C_4H_9-$ | $n_D^{23}$ 1.5773 |
| 8 | H— | H— | $-CH_2-$ | sec-$C_4H_9-$ | $n_D^{23}$ 1.5703 |
| 9 | H— | H— | $-CH_2-$ | $CH_2=CHCH_2-$ | mp 47-48°C |
| 10 | H— | H— | $-CH_2-$ | $HC\equiv CCH_2-$ | mp 52-53°C |
| 11 | H— | H— | $-CH_2-$ | 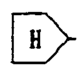 | $n_D^{23}$ 1.5704 |
| 12 | H— | H— | $-CH_2-$ | 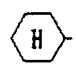 | $n_D^{23}$ 1.5751 |
| 13 | H— | H— | $-CH_2-$ | $CH_3OCH_2CH_2-$ | $n_D^{23}$ 1.5739 |
| 14 | H— | H— | $-CH_2-$ | 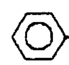 $CH_2-$ | $n_D^{23}$ 1.5760 |
| 15 | H— | H— | $-CH-$<br>$CH_3$ | i-$C_3H_7-$ | mp 72-73°C |
| 16 | H— | H— | $-(CH_2)_2-$ | i-$C_3H_7-$ | mp 96-97.5°C |
| 17 | H— | H— | $-(CH_2)_2-$ | $CH_3SCH_2CH_2-$ | $n_D^{23}$ 1.5741 |
| 18 | H— | H— | $-(CH_2)_3-$ | i-$C_3H_7-$ | $n_D^{23}$ 1.5782 |
| 19 | H— | 4'-Cl— | $-CH_2-$ | i-$C_5H_{11}-$ | $n_D^{23}$ 1.5872 |
| 20 | H— | 5'-Cl— | $-CH_2-$ | H— | $n_D^{23}$ 1.5731 |
| 21 | H— | 5'-Cl— | $-CH_2-$ | i-$C_3H_7-$ | mp 88-90°C |
| 22 | H— | 5'-Cl— | $-CH_2-$ | sec-$C_4H_9-$ | $n_D^{23}$ 1.5568 |

Table 1 (Continued-1)

| Compound No. | $X_m$ | $Y_n$ | A | R | Physical data |
|---|---|---|---|---|---|
| 23 | H— | 5'-Cl— | $-CH_2-$ | $HC{\equiv}CCH_2-$ | mp 94-95°C |
| 24 | H— | 5'-Cl— | $-CH_2-$ | $NCCH_2-$ | $n_D^{23}$ 1.5766 |
| 25 | H— | 5'-Cl— | $-CH(CH_3)-$ | $i\text{-}C_3H_7-$ | $n_D^{23}$ 1.5745 |
| 26 | H— | 5'-Cl— | $-(CH_2)_2-$ | $i\text{-}C_3H_7-$ | $n_D^{23}$ 1.5791 |
| 27 | H— | 5'-Cl— | $-CH(C_2H_5)-$ | $CH_3-$ | $n_D^{23}$ 1.5842 |
| 28 | H— | 6'-Cl— | $-CH_2-$ | $i\text{-}C_3H_7-$ | mp 94-95°C |
| 29 | H— | 6'-Cl— | $-CH_2-$ | $sec\text{-}C_4H_9-$ | $n_D^{23}$ 1.5785 |
| 30 | H— | 6'-Cl— | $-CH_2-$ | $HC{\equiv}CCH_2-$ | mp 75-77°C |
| 31 | H— | 6'-Cl— | $-CH_2-$ | $C_2H_5OCOCH_2-$ | $n_D^{23}$ 1.5726 |
| 32 | H— | 6'-Cl— | $-CH(CH_3)-$ | $i\text{-}C_3H_7-$ | $n_D^{23}$ 1.5721 |
| 33 | H— | 6'-Cl— | $-(CH_2)_2-$ | $i\text{-}C_3H_7-$ | $n_D^{23}$ 1.5745 |
| 34 | H— | 6'-Cl— | $-CH(C_3H_7\text{-}n)-$ | $n\text{-}C_4H_9-$ | $n_D^{23}$ 1.5743 |
| 35 | H— | 7'-Cl— | $-CH_2-$ | $C_2H_5-$ | $n_D^{23}$ 1.5702 |
| 36 | H— | 7'-Cl— | $-CH_2-$ | $n\text{-}C_4H_9OCH_2CH_2-$ | $n_D^{23}$ 1.5751 |
| 37 | H— | 5'-F— | $-CH_2-$ | $i\text{-}C_3H_7-$ | $n_D^{23}$ 1.5698 |
| 38 | H— | 5'-F— | $-CH_2-$ | $i\text{-}C_4H_9-$ | $n_D^{23}$ 1.5643 |
| 39 | H— | 5'-F— | $-CH(CH_3)-$ | $i\text{-}C_3H_7-$ | $n_D^{23}$ 1.5709 |
| 40 | H— | 6'-F— | $-CH_2-$ | H— | $n_D^{23}$ 1.5636 |
| 41 | H— | 6'-F— | $-CH_2-$ | $i\text{-}C_3H_7-$ | $n_D^{23}$ 1.5498 |
| 42 | H— | 6'-F— | $-CH_2-$ | $i\text{-}C_4H_9-$ | $n_D^{23}$ 1.5541 |
| 43 | H— | 6'-F— | $-CH_2-$ | $CH_2{=}C(CH_3)\text{-}CH_2-$ | $n_D^{23}$ 1.5576 |
| 44 | H— | 5'-Br— | $-CH_2-$ | $n\text{-}C_3H_7-$ | $n_D^{23}$ 1.5594 |
| 45 | H— | 5'-Br— | $-CH_2-$ | $i\text{-}C_3H_7-$ | $n_D^{23}$ 1.5771 |

Table 1 (Continued-2)

| Compound No. | $X_m$ | $Y_n$ | A | R | Physical data |
|---|---|---|---|---|---|
| 46 | H— | 5'-Br— | $-CH_2-$ | $C_2H_5SCH_2CH_2-$ | $n_D^{23}$ 1.5462 |
| 47 | H— | 6'-Br— | $-CH_2-$ | $i\text{-}C_3H_7-$ | mp 101-103°C |
| 48 | H— | 6'-Br— | $-CH_2-$ | $n\text{-}C_6H_{13}-$ | $n_D^{23}$ 1.5773 |
| 49 | H— | 5'-$CH_3$— | $-CH_2-$ | H— | mp 106-107.5°C |
| 50 | H— | 5'-$CH_3$— | $-CH_2-$ | $CH_3-$ | mp 92-93°C |
| 51 | H— | 5'-$CH_3$— | $-CH_2-$ | $C_2H_5-$ | mp 72-74°C |
| 52 | H— | 5'-$CH_3$— | $-CH_2-$ | $n\text{-}C_3H_7-$ | $n_D^{23}$ 1.5695 |
| 53 | H— | 5'-$CH_3$— | $-CH_2-$ | $i\text{-}C_3H_7-$ | mp 85-86°C |
| 54 | H— | 5'-$CH_3$— | $-CH_2-$ | $n\text{-}C_4H_9-$ | mp 72-73°C |
| 55 | H— | 5'-$CH_3$— | $-CH_2-$ | $i\text{-}C_4H_9-$ | $n_D^{23}$ 1.5742 |
| 56 | H— | 5'-$CH_3$— | $-CH_2-$ | $sec\text{-}C_4H_9-$ | $n_D^{23}$ 1.5688 |
| 57 | H— | 5'-$CH_3$— | $-CH_2-$ | $CH_2{=}CHCH_2-$ | $n_D^{23}$ 1.5711 |
| 58 | H— | 5'-$CH_3$— | $-CH_2-$ | 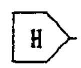 | $n_D^{23}$ 1.5764 |
| 59 | H— | 5'-$CH_3$— | $-CH_2-$ | 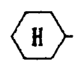 | $n_D^{23}$ 1.5713 |
| 60 | H— | 5'-$CH_3$— | $-CH_2-$ | $CH_3OCOCH_2-$ | $n_D^{23}$ 1.5668 |
| 61 | H— | 5'-$CH_3$— | $-CH_2-$ | 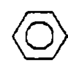 $CH_2-$ | $n_D^{23}$ 1.5749 |
| 62 | H— | 5'-$CH_3$— | $-CH(CH_3)-$ | $i\text{-}C_3H_7-$ | $n_D^{23}$ 1.5804 |
| 63 | H— | 5'-$CH_3$— | $-(CH_2)_2-$ | $i\text{-}C_3H_7-$ | mp 77-79°C |
| 64 | H— | 6'-$CH_3$— | $-CH_2-$ | $C_2H_5-$ | $n_D^{23}$ 1.5694 |
| 65 | H— | 6'-$CH_3$— | $-CH_2-$ | $i\text{-}C_3H_7-$ | mp 85-86°C |
| 66 | H— | 6'-$CH_3$— | $-CH_2-$ | $i\text{-}C_4H_9-$ | $n_D^{23}$ 1.5704 |
| 67 | H— | 6'-$CH_3$— | $-CH_2-$ | 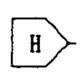 | $n_D^{23}$ 1.5687 |
| 68 | H— | 6'-$CH_3$— | $-CH_2-$ | $CH_3COCH_2-$ | $n_D^{23}$ 1.5704 |
| 69 | H— | 6'-$CH_3$— | $-(CH_2)_2-$ | $i\text{-}C_3H_7-$ | $n_D^{23}$ 1.5746 |

Table 1 (Continued-3)

| Compound No. | $X_m$ | $Y_n$ | A | R | Physical data |
|---|---|---|---|---|---|
| 70 | H— | 5'-$C_2H_5$— | —$CH_2$— | i-$C_3H_7$— | $n_D^{23}$ 1.5768 |
| 71 | H— | 5'-$C_2H_5$— | —$CH_2$— | i-$C_4H_9$— | $n_D^{23}$ 1.5720 |
| 72 | H— | 6'-$C_2H_5$— | —$CH_2$— | i-$C_3H_7$— | $n_D^{23}$ 1.5746 |
| 73 | H— | 5'-n-$C_3H_7$— | —$CH_2$— | i-$C_3H_7$— | $n_D^{23}$ 1.5715 |
| 74 | H— | 6'-n-$C_3H_7$— | —$CH_2$— | i-$C_3H_7$— | $n_D^{23}$ 1.5695 |
| 75 | H— | 5'-i-$C_3H_7$— | —$CH_2$— | i-$C_3H_7$— | $n_D^{23}$ 1.5708 |
| 76 | H— | 5'-i-$C_3H_7$— | —$CH_2$— | i-$C_4H_9$— | mp 80-82°C |
| 77 | H— | 6'-i-$C_3H_7$— | —$CH_2$— | i-$C_3H_7$— | $n_D^{23}$ 1.5741 |
| 78 | H— | 5'-n-$C_4H_9$— | —$CH_2$— | i-$C_3H_7$— | $n_D^{23}$ 1.5739 |
| 79 | H— | 5'-$CH_3O$— | —$CH_2$— | i-$C_3H_7$— | $n_D^{23}$ 1.5687 |
| 80 | H— | 5'-$CH_3O$— | —$CH_2$— | i-$C_3H_7$— | $n_D^{23}$ 1.5642 |
| 81 | H— | 5'—$C_2H_5O$— | —$CH_2$— | i-$C_3H_7$— | $n_D^{23}$ 1.5719 |
| 82 | H— | 5'-$C_2H_5O$— | —$CH_2$— | i-$C_3H_7$— | $n_D^{23}$ 1.5738 |
| 83 | H— | 5'-i-$C_3H_7O$— | —$CH_2$— | i-$C_3H_7$— | $n_D^{23}$ 1.5714 |
| 84 | H— | 5'-$CF_3$— | —$CH_2$— | i-$C_3H_7$— | $n_D^{23}$ 1.5688 |
| 85 | H— | 5'-$CF_3$— | —$CH_2$— | sec-$C_4H_9$— | $n_D^{23}$ 1.5683 |
| 86 | H— | 6'-$CF_3$— | —$CH_2$— | i-$C_3H_7$— | $n_D^{23}$ 1.5704 |
| 87 | H— | 6'-$CF_3$— | —$CH_2$— | $CH_2$=C($CH_3$)-$CH_2$— | $n_D^{23}$ 1.5711 |
| 88 | H— | 5'-$CHF_2O$— | —$CH_2$— | i-$C_3H_7$— | $n_D^{23}$ 1.5708 |
| 89 | H— | 6'-$CHF_2O$— | —$CH_2$— | i-$C_3H_7$— | $n_D^{23}$ 1.5743 |
| 90 | H— | 6'-$CF_3O$— | —$CH_2$— | i-$C_3H_7$— | $n_D^{23}$ 1.5692 |
| 91 | H— | 5'-$CH_3S$— | —$CH_2$— | i-$C_3H_7$— | $n_D^{23}$ 1.5688 |
| 92 | H— | 5'-$C_2H_5S$— | —$CH_2$— | i-$C_3H_7$— | $n_D^{23}$ 1.5715 |
| 93 | H— | 6'-i-$C_3H_7S$— | —$CH_2$— | i-$C_3H_7$— | $n_D^{23}$ 1.5639 |
| 94 | H— | 5'-$CH_3CO$— | —$CH_2$— | i-$C_3H_7$— | $n_D^{23}$ 1.5843 |
| 95 | H— | 5'-$NO_2$— | —$CH_2$— | i-$C_3H_7$— | mp 100-102°C |
| 96 | H— | 6'-$NO_2$— | —$CH_2$— | i-$C_3H_7$— | mp 105-108°C |

Table 1 (Continued-4)

| Compound No. | $X_m$ | $Y_n$ | A | R | Physical data |
|---|---|---|---|---|---|
| 97 | H− | 5′-CN− | $-CH_2-$ | $i\text{-}C_3H_7-$ | $n_D^{23}$ 1.5744 |
| 98 | H− | 5′-Cl-6′-$CH_3$− | $-CH_2-$ | $i\text{-}C_3H_7-$ | $n_D^{23}$ 1.5770 |
| 99 | H− | 5′-Cl-6′-$CH_3O$− | $-CH_2-$ | $i\text{-}C_3H_7-$ | $n_D^{23}$ 1.5635 |
| 100 | H− | 5′-Cl-6′-$CF_3$− | $-CH_2-$ | $i\text{-}C_3H_7-$ | $n_D^{23}$ 1.5697 |
| 101 | H− | 5′-$CH_3S$-6′-$CH_3$− | $-CH_2-$ | $i\text{-}C_3H_7-$ | $n_D^{23}$ 1.5497 |
| 102 | H− | 5′-$NO_2$-6′-Cl− | $-CH_2-$ | $i\text{-}C_3H_7-$ | $n_D^{23}$ 1.5772 |
| 103 | H− | 5′-CN-6′-Cl− | $-CH_2-$ | $i\text{-}C_3H_7-$ | $n_D^{23}$ 1.5706 |
| 104 | 2-Cl− | H− | $-CH_2-$ | $C_2H_5-$ | $n_D^{23}$ 1.5714 |
| 105 | 2-Cl− | H− | $-CH_2-$ | $i\text{-}C_3H_7-$ | mp 45-47°C |
| 106 | 2-Cl− | H− | $-CH_2-$ | $HC\equiv C\text{-}CH(CH_3)-$ | $n_D^{23}$ 1.5686 |
| 107 | 2-Cl− | H− | $-CH_2-$ | $n\text{-}C_3H_7OCH_2CH_2-$ | $n_D^{23}$ 1.5712 |
| 108 | 2-Cl− | 5′-Cl− | $-CH_2-$ | $i\text{-}C_3H_7-$ | mp 69-71°C |
| 109 | 2-Cl− | 5′-Cl− | $-CH(CH_3)-$ | $C_2H_5-$ | $n_D^{23}$ 1.5743 |
| 110 | 2-Cl− | 6′-Cl− | $-CH_2-$ | $i\text{-}C_3H_7-$ | $n_D^{23}$ 1.5683 |
| 111 | 2-Cl− | 6′-Cl− | $-CH(CH_3)-$ | 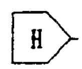 | $n_D^{23}$ 1.5699 |
| 112 | 2-Cl− | 6′-F− | $-CH_2-$ | $i\text{-}C_3H_7-$ | $n_D^{23}$ 1.5741 |
| 113 | 2-Cl− | 5′-$CH_3$− | $-CH_2-$ | $i\text{-}C_3H_7-$ | mp 83-85°C |
| 114 | 2-Cl− | 5′-$CH_3$− | $-CH_2-$ | $i\text{-}C_3H_7-$ | $n_D^{23}$ 1.5733 |
| 115 | 2-Cl− | 5′-$CH_3O$− | $-CH_2-$ | $CH_2{=}CHCH_2-$ | $n_D^{23}$ 1.5708 |
| 116 | 2-Cl− | 6′-$CF_3$− | $-CH_2-$ | $CH_3C\equiv CCH_2-$ | $n_D^{23}$ 1.5693 |
| 117 | 2-Cl− | 5′-$CHF_2O$− | $-CH_2-$ | $i\text{-}C_3H_7-$ | $n_D^{23}$ 1.5730 |
| 118 | 3-Cl− | H− | $-CH_2-$ | $i\text{-}C_3H_7-$ | $n_D^{23}$ 1.5498 |
| 119 | 3-Cl− | H− | $-(CH_2)_2-$ | $i\text{-}C_3H_7-$ | $n_D^{23}$ 1.5551 |
| 120 | 3-Cl− | 5′-Cl− | $-CH_2-$ | $i\text{-}C_3H_7-$ | mp 70-72°C |
| 121 | 3-Cl− | 6′-Cl− | $-CH_2-$ | $i\text{-}C_3H_7-$ | $n_D^{23}$ 1.5583 |

Table 1 (Continued-5)

| Compound No. | $X_m$ | $Y_n$ | A | R | Physical data |
|---|---|---|---|---|---|
| 122 | 3-Cℓ— | 5'-$CH_3$— | —$CH_2$— | i-$C_3H_7$— | mp 85-87°C |
| 123 | 3-Cℓ— | 6'-$CH_3$— | —$CH_2$— | i-$C_3H_7$— | $n_D^{23}$ 1.5641 |
| 124 | 3-Cℓ— | 5'-Cℓ-6'-$C_2H_5$— | —$CH_2$— | i-$C_3H_7$— | $n_D^{23}$ 1.5687 |
| 125 | 4-Cℓ— | H— | —$CH_2$— | $CH_3$— | $n_D^{23}$ 1.5580 |
| 126 | 4-Cℓ— | H— | —$CH_2$— | i-$C_3H_7$— | mp 87-88°C |
| 127 | 4-Cℓ— | H— | —$CH_2$— | i-$C_4H_9$— | $n_D^{23}$ 1.5648 |
| 128 | 4-Cℓ— | H— | —$CH_2$— | sec-$C_4H_9$— | mp 48-50°C |
| 129 | 4-Cℓ— | H— | —$CH_2$— | HC≡$CCH_2$— | mp 98-99°C |
| 130 | 4-Cℓ— | H— | —$CH_2$— | 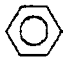$CH_2CH_2$— | $n_D^{23}$ 1.5746 |
| 131 | 4-Cℓ— | H— | —CH—<br>\|<br>$CH_3$ | i-$C_3H_7$— | $n_D^{23}$ 1.5816 |
| 132 | 4-Cℓ— | H— | $-(CH_2)_3-$ | i-$C_3H_7$— | $n_D^{23}$ 1.5759 |
| 133 | 4-Cℓ— | 5'-Cℓ— | —$CH_2$— | i-$C_3H_7$— | $n_D^{23}$ 1.5856 |
| 134 | 4-Cℓ— | 5'-Cℓ— | —$CH_2$— | $CH_2$=$CHCH_2$— | $n_D^{23}$ 1.5724 |
| 135 | 4-Cℓ— | 5'-Cℓ— | —$CH_2$— | HC≡$CCH_2$— | mp 139-140°C |
| 136 | 4-Cℓ— | 5'-Cℓ— | —$CH_2$— | $NCCH_2CH_2$— | $n_D^{23}$ 1.5744 |
| 137 | 4-Cℓ— | 5'-Cℓ— | —CH—<br>\|<br>$CH_3$ | i-$C_3H_7$— | $n_D^{23}$ 1.5844 |
| 138 | 4-Cℓ— | 6'-Cℓ— | —$CH_2$— | i-$C_3H_7$— | mp 107-110°C |
| 139 | 4-Cℓ— | 6'-Cℓ— | —$CH_2$— | sec-$C_4H_9$— | $n_D^{23}$ 1.5642 |
| 140 | 4-Cℓ— | 6'-Cℓ— | —$CH_2$— | HC≡$CCH_2$— | mp 94-95°C |
| 141 | 4-Cℓ— | 6'-Cℓ— | —$CH_2$— | $CH_3SCH$-$CH_2$—<br>\|<br>$CH_3$ | $n_D^{23}$ 1.5713 |
| 142 | 4-Cℓ— | 4'-F— | —$CH_2$— | i-$C_3H_7$— | $n_D^{23}$ 1.5658 |
| 143 | 4-Cℓ— | 5'-F— | —$CH_2$— | i-$C_3H_7$— | $n_D^{23}$ 1.5831 |
| 144 | 4-Cℓ— | 5'-F— | —$CH_2$— | i-$C_4H_9$— | $n_D^{23}$ 1.5811 |
| 145 | 4-Cℓ— | 5'-F— | —$CH_2$— | sec-$C_4H_9$— | $n_D^{23}$ 1.5734 |
| 146 | 4-Cℓ— | 5'-F— | —$CH_2$— | i-$C_4H_9$— | $n_D^{23}$ 1.5836 |

Table 1 (Continued-6)

| Compound No. | $X_m$ | $Y_n$ | A | R | Physical data |
|---|---|---|---|---|---|
| 147 | 4-Cl— | 6'-F— | —$CH_2$— | 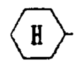 | $n_D^{23}$ 1.5638 |
| 148 | 4-Cl— | 5'-Br— | —$CH_2$— | i-$C_3H_7$— | $n_D^{23}$ 1.5781 |
| 149 | 4-Cl— | 6'-Br— | —$CH_2$— | i-$C_3H_7$— | mp 129-131°C |
| 150 | 4-Cl— | 4'-$CH_3$— | —$CH_2$— | i-$C_3H_7$— | $n_D^{23}$ 1.5714 |
| 151 | 4-Cl— | 5'-$CH_3$— | —$CH_2$— | i-$C_3H_7$— | $n_D^{23}$ 1.5649 |
| 152 | 4-Cl— | 5'-$CH_3$— | —$CH_2$— | i-$C_4H_9$— | $n_D^{23}$ 1.5640 |
| 153 | 4-Cl— | 6'-$CH_3$— | —$CH_2$— | i-$C_3H_7$— | mp 87-89°C |
| 154 | 4-Cl— | 6'-$CH_3$— | —$CH_2$— | i-$C_4H_9$— | $n_D^{23}$ 1.5625 |
| 155 | 4-Cl— | 7'-$CH_3$— | —$CH_2$— | i-$C_3H_7$— | $n_D^{23}$ 1.5713 |
| 156 | 4-Cl— | 5'-$C_2H_5$— | —$CH_2$— | i-$C_4H_9$— | $n_D^{23}$ 1.5694 |
| 157 | 4-Cl— | 5'-i-$C_3H_7$— | —$CH_2$— | i-$C_4H_9$— | $n_D^{23}$ 1.5716 |
| 158 | 4-Cl— | 5'-$CH_3O$— | —$CH_2$— | i-$C_3H_7$— | $n_D^{23}$ 1.5681 |
| 159 | 4-Cl— | 6'-$CF_3$— | —$CH_2$— | i-$C_3H_7$— | $n_D^{23}$ 1.5681 |
| 160 | 4-Cl— | 5'-$CHF_2O$— | —$CH_2$— | i-$C_3H_7$— | $n_D^{23}$ 1.5716 |
| 161 | 4-Cl— | 5'-$CH_3S$— | —$CH_2$— | i-$C_3H_7$— | $n_D^{23}$ 1.5795 |
| 162 | 4-Cl— | 5'-$CH_3CO$— | —$CH_2$— | i-$C_3H_7$— | $n_D^{23}$ 1.5722 |
| 163 | 4-Cl— | 5'-$NO_2$— | —$CH_2$— | i-$C_3H_7$— | mp 98-100°C |
| 164 | 4-Cl— | 6'-$NO_2$— | —$CH_2$— | i-$C_3H_7$— | mp 132-135°C |
| 165 | 2-F— | H— | —$CH_2$— | i-$C_3H_7$— | mp 54-56°C |
| 166 | 2-F— | 5'-Cl— | —$CH_2$— | i-$C_3H_7$— | mp 102-105°C |
| 167 | 2-F— | 6'-Cl— | —$CH_2$— | i-$C_3H_7$— | $n_D^{23}$ 1.5841 |
| 168 | 2-F— | 5'-$CH_3$— | —$CH_2$— | i-$C_3H_7$— | mp 112.5-114°C |
| 169 | 2-F— | 5'6'-$(CH_3)_2$ | $-(CH_2)_2-$ | n-$C_5H_{11}$— | $n_D^{23}$ 1.5714 |
| 170 | 3-F— | H— | —$CH_2$— | i-$C_3H_7$— | $n_D^{23}$ 1.5763 |
| 171 | 3-F— | 4'-Cl— | —$CH_2$— | i-$C_3H_7$— | $n_D^{23}$ 1.5775 |
| 172 | 3-F— | 5'-Cl— | —$CH_2$— | i-$C_3H_7$— | mp 87-89°C |
| 173 | 3-F— | 6'-Cl— | —$CH_2$— | i-$C_3H_7$— | $n_D^{23}$ 1.5814 |
| 174 | 3-F— | 5'-$CH_3$— | —$CH_2$— | i-$C_3H_7$— | mp 73-75°C |

Table 1 (Continued-7)

| Compound No. | $X_m$ | $Y_n$ | A | R | Physical data |
|---|---|---|---|---|---|
| 175 | 4-F- | H- | $-CH_2-$ | H- | mp 70-72°C |
| 176 | 4-F- | H- | $-CH_2-$ | $i-C_3H_7-$ | mp 80-90°C |
| 177 | 4-F- | H- | $-CH_2-$ | $s-C_4H_9-$ | $n_D^{23}$ 1.5903 |
| 178 | 4-F- | H- | $-CH_2-$ | $HC\equiv CCH_2-$ | mp 76-77°C |
| 179 | 4-F- | H- | $-CH_2-$ | $CH_3OCH_2CH_2-$ | $n_D^{23}$ 1.5643 |
| 180 | 4-F- | H- | $-CH-$<br>\|<br>$CH_3$ | $i-C_3H_7-$ | $n_D^{23}$ 1.5822 |
| 181 | 4-F- | H- | $-(CH_2)_2-$ | $n-C_3H_7-$ | $n_D^{23}$ 1.5763 |
| 182 | 4-F- | 5'-Cl- | $-CH_2-$ | $i-C_3H_7-$ | $n_D^{23}$ 1.5734 |
| 183 | 4-F- | 5'-Cl- | $-CH_2-$ | $HC\equiv CCH_2-$ | $n_D^{23}$ 1.5779 |
| 184 | 4-F- | 6'-Cl- | $-CH_2-$ | $i-C_3H_7-$ | mp 88-91°C |
| 185 | 4-F- | 6'-Cl- | $-CH_2-$ | $s-C_4H_9-$ | $n_D^{23}$ 1.5783 |
| 186 | 4-F- | 6'-Cl- | $-CH_2-$ | $HC\equiv CCH_2-$ | $n_D^{23}$ 1.5559 |
| 187 | 4-F- | 5'-F- | $-CH_2-$ | $i-C_3H_7-$ | $n_D^{23}$ 1.5678 |
| 188 | 4-F- | 6'-F- | $-CH_2-$ | $i-C_3H_7-$ | $n_D^{23}$ 1.5497 |
| 189 | 4-F- | 6'-Br- | $-(CH_2)_3-$ | $CH_3-$ | $n_D^{23}$ 1.5492 |
| 190 | 4-F- | $5'-CH_3-$ | $-CH_2-$ | $i-C_3H_7-$ | mp 97-99°C |
| 191 | 4-F- | $6'-CH_3-$ | $-CH_2-$ | $i-C_3H_7-$ | $n_D^{23}$ 1.5722 |
| 192 | 2-Br- | H- | $-CH_2-$ | $i-C_3H_7-$ | $n_D^{23}$ 1.5599 |
| 193 | 3-Br- | 5'-Cl- | $-CH_2-$ | $i-C_3H_7-$ | $n_D^{23}$ 1.5692 |
| 194 | 4-Br- | H- | $-CH_2-$ | $i-C_3H_7-$ | mp 46-48°C |
| 195 | 4-Br- | H- | $-CH_2-$ | 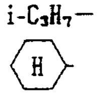 | $n_D^{23}$ 1.5736 |
| 196 | 4-Br- | 5'-Cl- | $-CH_2-$ | $i-C_3H_7-$ | mp 87-89°C |
| 197 | 4-Br- | 5'-Cl- | $-CH_2-$ | $s-C_4H_9-$ | $n_D^{23}$ 1.5814 |
| 198 | 4-Br- | 5'-F- | $-CH_2-$ | $i-C_3H_7-$ | $n_D^{23}$ 1.5699 |
| 199 | 4-Br- | 6'-F- | $-CH_2-$ | $i-C_3H_7-$ | $n_D^{23}$ 1.5714 |
| 200 | 4-Br- | $5'-CH_3-$ | $-CH_2-$ | $i-C_3H_7-$ | mp 63-65°C |
| 201 | $2-CH_3-$ | H- | $-CH_2-$ | $C_2H_5-$ | $n_D^{23}$ 1.5659 |

Table 1 (Continued-8)

| Compound No. | $X_m$ | $Y_n$ | A | R | Physical data |
|---|---|---|---|---|---|
| 202 | 2-$CH_3$— | H— | —$CH_2$— | i-$C_3H_7$— | mp 69.5-71℃ |
| 203 | 2-$CH_3$— | H— | —$CH_2$— | i-$C_4H_9$— | mp 105-106℃ |
| 204 | 2-$CH_3$— | 5'-Cℓ— | —$CH_2$— | i-$C_3H_7$— | $n_D^{23}$ 1.5569 |
| 205 | 2-$CH_3$— | 6'-Cℓ— | —$CH_2$— | i-$C_3H_7$— | mp 65-67℃ |
| 206 | 2-$CH_3$— | 5'-F— | —$CH_2$— | i-$C_3H_7$— | $n_D^{23}$ 1.5736 |
| 207 | 2-$CH_3$— | 5'-F— | —$CH_2$— | i-$C_4H_9$— | mp 68-70℃ |
| 208 | 2-$CH_3$— | 6'-F— | —$CH_2$— | i-$C_3H_7$— | mp 62-63℃ |
| 209 | 2-$CH_3$— | 6'-F— | —$CH_2$— | i-$C_4H_9$— | mp 90-91℃ |
| 210 | 2-$CH_3$— | 5'-$CH_3$— | —$CH_2$— | i-$C_3H_7$— | $n_D^{23}$ 1.5731 |
| 211 | 2-$CH_3$— | 5'-$CH_3$— | —$CH_2$— | i-$C_4H_9$— | $n_D^{23}$ 1.5749 |
| 212 | 2-$CH_3$— | 6'-$CH_3$— | —$CH_2$— | i-$C_3H_7$— | $n_D^{23}$ 1.5736 |
| 213 | 2-$CH_3$— | 6'-$CH_3$— | —$CH_2$— | i-$C_4H_9$— | $n_D^{23}$ 1.5668 |
| 214 | 2-$CH_3$— | 5'-$C_2H_5$— | —$CH_2$— | i-$C_3H_7$— | mp 67-70℃ |
| 215 | 2-$CH_3$— | 5'-$C_2H_5$— | —$CH_2$— | i-$C_4H_9$— | $n_D^{23}$ 1.5702 |
| 216 | 2-$CH_3$— | 5'-n-$C_3H_7$— | —$CH_2$— | i-$C_3H_7$— | mp 63-65℃ |
| 217 | 2-$CH_3$— | 5'-i-$C_3H_7$— | —$CH_2$— | i-$C_3H_7$— | mp 77-80℃ |
| 218 | 2-$CH_3$— | 5'-i-$C_3H_7$— | —$CH_2$— | i-$C_4H_9$— | $n_D^{23}$ 1.5744 |
| 219 | 2-$CH_3$— | 6'-n-$C_4H_9$— | —$CH_2$— | i-$C_3H_7$— | $n_D^{23}$ 1.5703 |
| 220 | 2-$CH_3$— | 5'-$CH_3O$— | —$CH_2$— | i-$C_3H_7$— | $n_D^{23}$ 1.5736 |
| 221 | 2-$CH_3$— | 6'-$CH_3O$— | —$CH_2$— | i-$C_3H_7$— | $n_D^{23}$ 1.5694 |
| 222 | 2-$CH_3$— | 5'-$C_2H_5O$— | —$CH_2$— | i-$C_3H_7$— | $n_D^{23}$ 1.5675 |
| 223 | 2-$CH_3$— | 5'-$CF_3$— | —$CH_2$— | i-$C_3H_7$— | $n_D^{23}$ 1.5683 |
| 224 | 2-$CH_3$— | 6'-$CF_2O$— | —$CH_2$— | i-$C_3H_7$— | $n_D^{23}$ 1.5701 |
| 225 | 2-$CH_3$— | 5'-$CF_3S$— | —$CH_2$— | i-$C_3H_7$— | $n_D^{23}$ 1.5716 |
| 226 | 2-$CH_3$— | 5'-$CH_3CO$-6'-Cℓ— | —$CH_2$— | i-$C_3H_7$— | $n_D^{23}$ 1.5721 |
| 227 | 3-$CH_3$— | H— | —$CH_2$— | i-$C_3H_7$— | $n_D^{23}$ 1.5575 |
| 228 | 3-$CH_3$— | 5'-Cℓ— | —$CH_2$— | i-$C_3H_7$— | $n_D^{23}$ 1.5605 |
| 229 | 3-$CH_3$— | 6'-Cℓ— | —$CH_2$— | i-$C_3H_7$— | $n_D^{23}$ 1.5602 |
| 230 | 3-$CH_3$— | 5'-$CH_3$— | —$CH_2$— | i-$C_3H_7$— | $n_D^{23}$ 1.5643 |

Table 1 (Continued-9)

| Compound No. | $X_m$ | $Y_n$ | A | R | Physical data |
|---|---|---|---|---|---|
| 231 | 3-$CH_3$— | 5'-n-$C_3H_7O$— | —$CH_2$— | $CH_2$=$CHCH_2$— | $n_D^{23}$ 1.5687 |
| 232 | 4-$CH_3$— | H— | —$CH_2$— | i-$C_3H_7$— | $n_D^{23}$ 1.5570 |
| 233 | 4-$CH_3$— | H— | —$CH_2$— | i-$C_4H_9$— | $n_D^{23}$ 1.5640 |
| 234 | 4-$CH_3$— | 5'-Cℓ— | —$CH_2$— | i-$C_3H_7$— | mp 74-76°C |
| 235 | 4-$CH_3$— | 6'-Cℓ— | —$CH_2$— | i-$C_3H_7$— | mp 98-100°C |
| 236 | 4-$CH_3$— | 5'-F— | —$CH_2$— | i-$C_4H_9$— | $n_D^{23}$ 1.5651 |
| 237 | 4-$CH_3$— | 6'-F— | —$CH_2$— | i-$C_4H_9$— | $n_D^{23}$ 1.5714 |
| 238 | 4-$CH_3$— | 5'-$CH_3$— | —$CH_2$— | i-$C_3H_7$— | $n_D^{23}$ 1.5642 |
| 239 | 4-$CH_3$— | 5'-$CH_3$— | —$CH_2$— | i-$C_4H_9$— | $n_D^{23}$ 1.5698 |
| 240 | 4-$CH_3$— | 6'-$CH_3$— | —$CH_2$— | i-$C_3H_7$— | mp 55-57°C |
| 241 | 4-$CH_3$— | 6'-$CH_3$— | —$CH_2$— | i-$C_4H_9$— | $n_D^{23}$ 1.5702 |
| 242 | 4-$CH_3$— | 5'-$C_2H_5$— | —$CH_2$— | i-$C_4H_9$— | $n_D^{23}$ 1.5731 |
| 243 | 4-$CH_3$— | 5'-i-$C_3H_7$— | —$CH_2$— | i-$C_4H_9$— | $n_D^{23}$ 1.5694 |
| 244 | 4-$CH_3$— | 5'-$CF_3$— | $-(CH_2)_2-$ | $C_2H_5$— | $n_D^{23}$ 1.5704 |
| 245 | 4-$CH_3$— | 5'-$C_2H_5$S-6'-$CH_3$- | —$CH_2$— | i-$C_3H_7$— | $n_D^{23}$ 1.5708 |
| 246 | 2-$C_2H_5$— | H— | —$CH_2$— | i-$C_3H_7$— | $n_D^{23}$ 1.5663 |
| 247 | 4-$C_2H_5$— | H— | —$CH_2$— | i-$C_3H_7$— | $n_D^{23}$ 1.5681 |
| 248 | 4-$C_2H_5$— | 5'-$CH_3$— | —$CH_2$— | i-$C_3H_7$— | $n_D^{23}$ 1.5684 |
| 249 | 4-$C_2H_5$— | 6'-$CH_3$— | —$CH_2$— | i-$C_3H_7$— | mp 80-83°C |
| 250 | 4-n-$C_3H_7$— | H— | —$CH_2$— | i-$C_3H_7$— | $n_D^{23}$ 1.5675 |
| 251 | 4-i-$C_3H_7$— | H— | —$CH_2$— | i-$C_3H_7$— | $n_D^{23}$ 1.5706 |
| 252 | 4-i-$C_3H_7$— | 5'-$CH_3$— | —$CH_2$— | i-$C_3H_7$— | $n_D^{23}$ 1.5693 |
| 253 | 4-i-$C_3H_7$— | 6'-$CH_3$— | —$CH_2$— | i-$C_3H_7$— | $n_D^{23}$ 1.5685 |
| 254 | 4-t-$C_4H_9$— | H— | —$CH_2$— | i-$C_3H_7$— | mp 91-93°C |
| 255 | 4-t-$C_4H_9$— | 5'-Cℓ— | —$CH_2$— | i-$C_3H_7$— | mp 97-98.5°C |
| 256 | 4-t-$C_4H_9$— | 6'-Cℓ— | —$CH_2$— | i-$C_3H_7$— | $n_D^{23}$ 1.5470 |
| 257 | 4-t-$C_4H_9$— | 5'-$CH_3$— | —$CH_2$— | i-$C_3H_7$— | $n_D^{23}$ 1.5649 |
| 258 | 4-t-$C_4H_9$— | 6'-$CH_3$— | —$CH_2$— | i-$C_3H_7$— | $n_D^{23}$ 1.5687 |
| 259 | 4-t-$C_4H_9$— | 5'-CN-6'-$C_2H_5$— | $-(CH_2)_3-$ | i-$C_3H_7$— | $n_D^{23}$ 1.5663 |

Table 1 (Continued-10)

| Compound No. | $X_m$ | $Y_n$ | A | R | Physical data |
|---|---|---|---|---|---|
| 260 | 2-$CH_3O$— | H— | —$CH_2$— | i-$C_3H_7$— | $n_D^{23}$ 1.5705 |
| 261 | 2-$CH_3O$— | 5'-Cℓ— | —$CH_2$— | i-$C_3H_7$— | $n_D^{23}$ 1.5691 |
| 262 | 2-$CH_3O$— | 5'-$CH_3$— | —$CH_2$— | i-$C_3H_7$— | $n_D^{23}$ 1.5687 |
| 263 | 3-$CH_3O$— | H— | —$CH_2$— | i-$C_3H_7$— | mp 60-63°C |
| 264 | 3-$CH_3O$— | 5'-Cℓ— | —$CH_2$— | i-$C_3H_7$— | mp 70-72°C |
| 265 | 3-$CH_3O$— | 5'-$CH_3$— | —$CH_2$— | i-$C_3H_7$— | mp 57-58.5°C |
| 266 | 4-$CH_3O$— | H— | —$CH_2$— | i-$C_3H_7$— | mp 57-60°C |
| 267 | 4-$CH_3O$— | 5'-Cℓ— | —$CH_2$— | i-$C_3H_7$— | mp 105-165.5°C |
| 268 | 4-$CH_3O$— | 6'-Cℓ— | —$CH_2$— | i-$C_3H_7$— | mp 58-61°C |
| 269 | 4-$CH_3O$— | 5'-$CH_3$— | —$CH_2$— | i-$C_3H_7$— | mp 57-60°C |
| 270 | 4-$CH_3O$— | 6'-$CH_3$— | —$CH_2$— | i-$C_3H_7$— | $n_D^{23}$ 1.5713 |
| 271 | 2-$C_2H_5O$— | H— | —$CH_2$— | i-$C_3H_7$— | $n_D^{23}$ 1.5687 |
| 272 | 2-$C_2H_5O$— | 5'-F— | —$CH_2$— | i-$C_3H_7$— | $n_D^{23}$ 1.5679 |
| 273 | 2-$C_2H_5O$— | 6'-F— | —$CH_2$— | i-$C_3H_7$— | $n_D^{23}$ 1.5712 |
| 274 | 2-$C_2H_5O$— | 5'-$CH_3$— | —$CH_2$— | i-$C_3H_7$— | $n_D^{23}$ 1.5694 |
| 275 | 2-$C_2H_5O$— | 6'-$CH_3$— | —$CH_2$— | i-$C_3H_7$— | $n_D^{23}$ 1.5688 |
| 276 | 4-$C_2H_5O$— | H— | —$CH_2$— | i-$C_3H_7$— | $n_D^{23}$ 1.5681 |
| 277 | 4-$C_2H_5O$— | 5'-$CH_3$— | —$CH_2$— | i-$C_3H_7$— | mp 95-96.5°C |
| 278 | 4-$C_2H_5O$— | 6'-$CH_3$— | —$CH_2$— | i-$C_3H_7$— | mp 100.5-102.5°C |
| 279 | 4-n-$C_3H_7O$- | 5',6'-$Cℓ_2$— | —$CH_2$— | i-$C_3H_7$— | $n_D^{23}$ 1.5711 |
| 280 | 2-i-$C_3H_7O$- | H— | —$CH_2$— | i-$C_3H_7$— | $n_D^{23}$ 1.5764 |
| 281 | 2-i-$C_3H_7O$- | 5'-$CH_3$— | —$CH_2$— | i-$C_3H_7$— | $n_D^{23}$ 1.5644 |
| 282 | 2-i-$C_3H_7O$- | 6'-$CH_3$— | —$CH_2$— | i-$C_3H_7$— | $n_D^{23}$ 1.5689 |
| 283 | 3-i-$C_3H_7O$- | H— | —$CH_2$— | i-$C_3H_7$— | $n_D^{23}$ 1.5672 |
| 284 | 3-i-$C_3H_7O$- | 5'-F— | —$CH_2$— | i-$C_3H_7$— | $n_D^{23}$ 1.5701 |
| 285 | 3-i-$C_3H_7O$- | 6'-F— | —$CH_2$— | i-$C_3H_7$— | $n_D^{23}$ 1.5685 |
| 286 | 3-i-$C_3H_7O$- | 5'-$CH_3$— | —$CH_2$— | i-$C_3H_7$— | $n_D^{23}$ 1.5671 |
| 287 | 3-i-$C_3H_7O$- | 6'-$CH_3$— | —$CH_2$— | i-$C_3H_7$— | $n_D^{23}$ 1.5740 |
| 288 | 4-i-$C_3H_7O$- | H— | —$CH_2$— | i-$C_3H_7$— | mp 102.5-103.5°C |

Table 1 (Continued-11)

| Compound No. | $X_m$ | $Y_n$ | A | R | Physical data |
|---|---|---|---|---|---|
| 289 | 4-i-$C_3H_7O$- | 5'-$CH_3$— | —$CH_2$— | i-$C_3H_7$— | $n_D^{23}$ 1.5691 |
| 290 | 4-i-$C_3H_7O$- | 6'-$CH_3$— | —$CH_2$— | i-$C_3H_7$— | mp 78.5-79.5°C |
| 291 | 2-$CF_3$— | H— | —$CH_2$— | i-$C_3H_7$— | mp 64-67°C |
| 292 | 2-$CF_3$— | 5'-Cl— | —$CH_2$— | i-$C_3H_7$— | $n_D^{23}$ 1.5723 |
| 293 | 2-$CF_3$— | 5'-$CH_3$— | —$CH_2$— | i-$C_3H_7$— | mp 75-77°C |
| 294 | 3-$CF_3$— | H— | —$CH_2$— | i-$C_3H_7$— | $n_D^{23}$ 1.5689 |
| 295 | 3-$CF_3$— | 5'-Cl— | —$CH_2$— | i-$C_3H_7$— | $n_D^{23}$ 1.5667 |
| 296 | 3-$CF_3$— | 5'-$CH_3$— | —$CH_2$— | i-$C_3H_7$— | $n_D^{23}$ 1.5669 |
| 297 | 4-$CF_3$— | H— | —$CH_2$— | i-$C_3H_7$— | mp 82-84°C |
| 298 | 4-$CF_3$— | H— | —CH—<br>\|<br>$CH_3$ | $C_2H_5$— | $n_D^{23}$ 1.5686 |
| 299 | 4-$CF_3$— | 5'-Cl— | —$CH_2$— | i-$C_3H_7$— | mp 103-105°C |
| 300 | 4-$CF_3$— | 5'-$CH_3$— | —$CH_2$— | i-$C_3H_7$— | mp 90-93°C |
| 301 | 2-$CHF_2O$— | H— | —$CH_2$— | i-$C_3H_7$— | $n_D^{23}$ 1.5691 |
| 302 | 2-$CHF_2O$— | 5'-$CH_3$— | —$CH_2$— | i-$C_3H_7$— | $n_D^{23}$ 1.5671 |
| 303 | 3-$CHF_2O$— | H— | —$CH_2$— | i-$C_3H_7$— | $n_D^{23}$ 1.5688 |
| 304 | 3-$CHF_2O$— | 5'-$CH_3$— | —$CH_2$— | i-$C_3H_7$— | $n_D^{23}$ 1.5674 |
| 305 | 4-$CHF_2O$— | H— | —$CH_2$— | i-$C_3H_7$— | mp 68-70°C |
| 306 | 4-$CHF_2O$— | 5'-$CH_3$— | —$CH_2$— | i-$C_3H_7$— | $n_D^{23}$ 1.5701 |
| 307 | 4-$CHF_2O$— | 6'-$C_2H_5O$— | $-(CH_2)_2-$ | HC≡C-CH—<br>\|<br>$CH_3$ | $n_D^{23}$ 1.5694 |
| 308 | 4-$CHF_2O$— | 5'-$CH_3S$— | —$CH_2$— | i-$C_3H_7$— | $n_D^{23}$ 1.5723 |
| 309 | 4-$CF_3O$— | H— | —$CH_2$— | i-$C_3H_7$— | $n_D^{23}$ 1.5765 |
| 310 | 4-$CF_3O$— | 5'-$CH_3$— | —$CH_2$— | i-$C_3H_7$— | $n_D^{23}$ 1.5673 |
| 311 | 4-$CF_3O$— | 5',6'-$Cl_2$— | —$CH_2$— | i-$C_3H_7$— | $n_D^{23}$ 1.5667 |
| 312 | 2-$CF_3S$— | H— | —$CH_2$— | i-$C_3H_7$— | $n_D^{23}$ 1.5734 |
| 313 | 2-$CH_3S$— | 5'-$CH_3$— | —$CH_2$— | i-$C_3H_7$— | $n_D^{23}$ 1.5713 |
| 314 | 3-$CF_3S$— | H— | —$CH_2$— | i-$C_3H_7$— | $n_D^{23}$ 1.5691 |
| 315 | 4-$CF_3S$— | H— | —$CH_2$— | i-$C_3H_7$— | $n_D^{23}$ 1.5704 |

Table 1 (Continued-12)

| Compound No. | $X_m$ | $Y_n$ | A | R | Physical data |
|---|---|---|---|---|---|
| 316 | 4-$CH_3S$— | 5'-Cl— | —$CH_2$— | i-$C_3H_7$— | $n_D^{23}$ 1.5683 |
| 317 | 4-$CH_3S$— | 6'-Cl— | —$CH_2$— | i-$C_3H_7$— | $n_D^{23}$ 1.5671 |
| 318 | 4-$CH_3S$— | 5'-$CH_3$— | —$CH_2$— | i-$C_3H_7$— | $n_D^{23}$ 1.5655 |
| 319 | 4-$CH_3S$— | 5'-$CH_3$— | $-(CH_2)_2-$ | i-$C_3H_7$— | $n_D^{23}$ 1.5746 |
| 320 | 4-$C_2H_5S$— | H— | —$CH_2$— | i-$C_3H_7$— | $n_D^{23}$ 1.5738 |
| 321 | 4-n-$C_3H_7S$- | H— | —$CH_2$— | i-$C_3H_7$— | $n_D^{23}$ 1.5668 |
| 322 | 4-$CH_3SO$— | H— | —$CH_2$— | i-$C_3H_7$— | $n_D^{23}$ 1.5723 |
| 323 | 4-$CH_3SO$— | 5'-Cl— | —$CH_2$— | i-$C_3H_7$— | $n_D^{23}$ 1.5743 |
| 324 | 4-$CH_3SO_2$— | H— | —$CH_2$— | i-$C_3H_7$— | $n_D^{23}$ 1.5732 |
| 325 | 4-$CH_3SO_2$— | 5'-Cl— | —$CH_2$— | i-$C_3H_7$— | $n_D^{23}$ 1.5647 |
| 326 | 2-$CH_3CO$— | H— | —$CH_2$— | i-$C_3H_7$— | $n_D^{23}$ 1.5673 |
| 327 | 2-$CH_3CO$— | 5'-Cl— | —$CH_2$— | i-$C_3H_7$— | $n_D^{23}$ 1.5647 |
| 328 | 3-$CH_3CO$— | H— | —$CH_2$— | i-$C_3H_7$— | $n_D^{23}$ 1.5681 |
| 329 | 4-$CH_3CO$— | H— | —$CH_2$— | i-$C_3H_7$— | $n_D^{23}$ 1.5718 |
| 330 | 4-$CH_3CO$— | 5'-Cl— | —$CH_2$— | i-$C_3H_7$— | $n_D^{23}$ 1.5686 |
| 331 | 4-$CH_3CO$— | 5'-Cl-6'-$C_2H_5$- | —$CH_2$— | i-$C_3H_7$— | $n_D^{23}$ 1.5688 |
| 332 | 4-$NO_2$— | H— | —$CH_2$— | i-$C_3H_7$— | $n_D^{23}$ 1.5716 |
| 333 | 4-$NO_2$— | 5'-Cl— | —$CH_2$— | i-$C_3H_7$— | $n_D^{23}$ 1.5733 |
| 334 | 4-$NO_2$— | 6'-$CH_3$— | —$CH_2$— | i-$C_3H_7$— | $n_D^{23}$ 1.5678 |
| 335 | 4-CN— | H— | —$CH_2$— | i-$C_3H_7$— | $n_D^{23}$ 1.5699 |
| 336 | 4-CN— | 5'-Cl— | —$CH_2$— | i-$C_3H_7$— | $n_D^{23}$ 1.5723 |
| 337 | 4-$C_2H_5OCO$- | H— | —$CH_2$— | i-$C_3H_7$— | $n_D^{23}$ 1.5726 |
| 338 | 4-$C_2H_5OCO$- | 5'-$CH_3$— | —$CH_2$— | i-$C_3H_7$— | $n_D^{23}$ 1.5738 |
| 339 | 2- 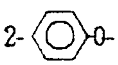 O- | H— | —$CH_2$— | $CH_3$— | $n_D^{23}$ 1.5687 |
| 340 | 2- 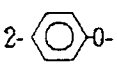 O- | H— | —$CH_2$— | i-$C_3H_7$— | $n_D^{23}$ 1.5669 |
| 341 | 3- 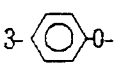 O- | H— | —$CH_2$— | $CH_3$— | $n_D^{23}$ 1.5719 |
| 342 | 3- 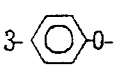 O- | H— | —$CH_2$— | $C_2H_5$— | mp 100-102°C |

Table 1 (Continued-13)

| Compound No. | $X_m$ | $Y_n$ | A | R | Physical data |
|---|---|---|---|---|---|
| 343 | 3- 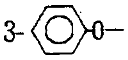 | H— | $—CH_2—$ | $n-C_3H_7—$ | $n_D^{23}$ 1.5658 |
| 344 | 3- 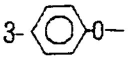 | H— | $—CH_2—$ | $i-C_3H_7—$ | $n_D^{23}$ 1.5689 |
| 345 | 3- 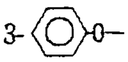 | 5'-Cℓ— | $—CH_2—$ | $i-C_3H_7—$ | $n_D^{23}$ 1.5717 |
| 346 | 3- 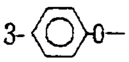 | 6'-Cℓ— | $—CH_2—$ | $i-C_3H_7—$ | $n_D^{23}$ 1.5693 |
| 347 | 3- 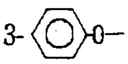 | 6'-Br— | $—CH_2—$ | $i-C_3H_7—$ | $n_D^{23}$ 1.5682 |
| 348 | 3- 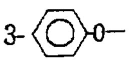 | 5'-$CH_3$— | $—CH_2—$ | $i-C_3H_7—$ | $n_D^{23}$ 1.5705 |
| 349 | 3- 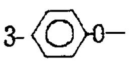 | 6'-$CH_3$— | $—CH_2—$ | $i-C_3H_7—$ | $n_D^{23}$ 1.5714 |
| 350 | 3- 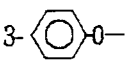 | 5'-$NO_2$— | $—CH_2—$ | $i-C_3H_7—$ | $n_D^{23}$ 1.5690 |
| 351 | 3- 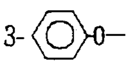 | 5'-$CH_3$-6'-F— | $—CH_2—$ | $i-C_3H_7—$ | $n_D^{23}$ 1.5711 |
| 352 | 4- 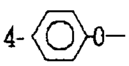 | H— | $—CH_2—$ | $CH_3—$ | $n_D^{23}$ 1.5724 |
| 353 | 4- 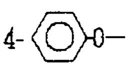 | H— | $—CH_2—$ | $i-C_3H_7—$ | $n_D^{23}$ 1.5748 |
| 354 | 4- 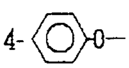 | H— | $-(CH_2)_3-$ | $C_2H_5—$ | $n_D^{23}$ 1.5725 |
| 355 | 4- 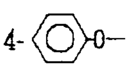 | 5'-Cℓ— | $—CH_2—$ | $i-C_3H_7—$ | $n_D^{23}$ 1.5714 |
| 356 | 4- 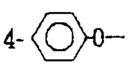 | 5'-$CH_3$— | $—CH_2—$ | $i-C_3H_7—$ | $n_D^{23}$ 1.5726 |
| 357 | 2- 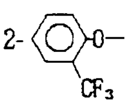 | H— | $—CH_2—$ | $i-C_3H_7—$ | $n_D^{23}$ 1.5695 |
| 358 | 3-Cℓ- 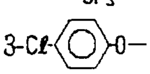 | H— | $—CH_2—$ | $i-C_3H_7—$ | $n_D^{23}$ 1.5704 |
| 359 | 3-F 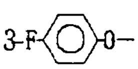 | H— | $—CH(CH_3)—$ | $CH_3—$ | mp 113-116°C |
| 360 | 3- 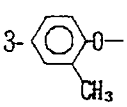 | H— | $—CH_2—$ | $i-C_3H_7—$ | $n_D^{23}$ 1.5751 |

Table 1 (Continued-14)

| Compound No. | $X_m$ | $Y_n$ | A | R | Physical data |
|---|---|---|---|---|---|
| 361 | 4-Cl-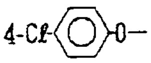-O- | H- | $-CH_2-$ | $i-C_3H_7-$ | $n_D^{23}$ 1.5720 |
| 362 | 4-F-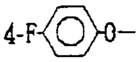-O- | H- | $-CH-$<br>$\vert$<br>$CH_3$ | $CH_3-$ | mp 107-109°C |
| 363 | $4-CH_3$-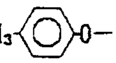-O- | H- | $-CH_2-$ | $i-C_3H_7-$ | $n_D^{23}$ 1.5693 |
| 364 | $4-t-C_4H_9$-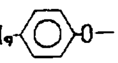-O- | H- | $-CH-$<br>$\vert$<br>$CH_3$ | $CH_3-$ | $n_D^{23}$ 1.5688 |
| 365 | $4-CH_3O$-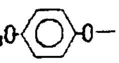-O- | H- | $-CH_2-$ | $i-C_3H_7-$ | $n_D^{23}$ 1.5705 |
| 366 | $2,4-Cl_2-$ | H- | $-CH_2-$ | $i-C_3H_7-$ | mp 80-82°C |
| 367 | $2,4-Cl_2-$ | $5'$-Cl- | $-CH_2-$ | $i-C_3H_7-$ | mp 95-97°C |
| 368 | $2,4-Cl_2-$ | $6'$-Cl- | $-CH_2-$ | $i-C_3H_7-$ | mp 52-55°C |
| 369 | $2,5-Cl_2-$ | $5',6'-Cl_2-$ | $-CH_2-$ | $i-C_3H_7-$ | $n_D^{23}$ 1.5740 |
| 370 | $3,4-Cl_2-$ | H- | $-CH_2-$ | $i-C_3H_7-$ | mp 90-92°C |
| 371 | $3,4-Cl_2-$ | $5'$-Cl- | $-CH_2-$ | $i-C_3H_7-$ | $n_D^{23}$ 1.5732 |
| 372 | $3,4-Cl_2-$ | $6'$-Cl- | $-CH_2-$ | $i-C_3H_7-$ | $n_D^{23}$ 1.5745 |
| 373 | 2-Cl-4-F- | H- | $-CH_2-$ | $i-C_3H_7-$ | $n_D^{23}$ 1.5736 |
| 374 | 2-Cl-4-F- | $5'-CH_3O-$ | $-CH-$<br>$\vert$<br>$C_2H_5$ | $n-C_5H_{11}-$ | $n_D^{23}$ 1.5730 |
| 375 | 2-Cl-4-Br- | H- | $-CH_2-$ | $i-C_3H_7-$ | $n_D^{23}$ 1.5711 |
| 376 | 3-Cl-4-Br- | $5'-NO_2-$ | $-CH-$<br>$\vert$<br>$CH_3$ | $C_2H_5-$ | $n_D^{23}$ 1.5692 |
| 377 | 2-Br-4-Cl- | H- | $-CH_2-$ | $i-C_3H_7-$ | $n_D^{23}$ 1.5688 |
| 378 | $2-Cl-4-CH_3-$ | H- | $-CH_2-$ | $i-C_3H_7-$ | $n_D^{23}$ 1.5674 |
| 379 | $2-Cl-4-CH_3-$ | $5'-C_2H_5O-$ | $-(CH_2)_2-$ | $n-C_3H_7-$ | $n_D^{23}$ 1.5709 |
| 380 | $2-CH_3-4-Cl-$ | $5'$-Cl-$6'$-F- | $-CH_2-$ | $i-C_3H_7-$ | $n_D^{23}$ 1.5744 |
| 381 | $2-CH_3O-4-Cl-$ | H- | $-CH_2-$ | $i-C_3H_7-$ | $n_D^{23}$ 1.5720 |
| 382 | $2-CH_3O-4-Cl-$ | $5'$-F- | $-CH_2-$ | $i-C_3H_7-$ | $n_D^{23}$ 1.5716 |
| 383 | $2-CF_3-4-Cl-$ | H- | $-CH_2-$ | $i-C_3H_7-$ | $n_D^{23}$ 1.5669 |
| 384 | $2-Cl-4-CHF_2O-$ | H- | $-CH_2-$ | $i-C_3H_7-$ | $n_D^{23}$ 1.5687 |

Table 1 (Continued-15)

| Compound No. | $X_m$ | $Y_n$ | A | R | Physical data |
|---|---|---|---|---|---|
| 385 | 2-Cℓ-4-$CH_3SO_2$− | H− | −$CH_2$− | i-$C_3H_7$− | $n_D^{23}$ 1.5694 |
| 386 | 2-Cℓ-4-$CH_3SO_2$− | 5'-Cℓ− | −$CH_2$− | i-$C_3H_7$− | $n_D^{23}$ 1.5681 |
| 387 | 2-Cℓ-4-$CH_3SO_2$− | 5'-$CH_3$− | −$CH_2$− | i-$C_3H_7$− | $n_D^{23}$ 1.5692 |
| 388 | 2-Cℓ-4-$NO_2$− | H− | −$CH_2$− | i-$C_3H_7$− | $n_D^{23}$ 1.5687 |
| 389 | 2-Cℓ-4-$NO_2$− | 5'-Cℓ− | −CH−(CH$_3$) | $CH_3OCH_2CH_2$− | $n_D^{23}$ 1.5705 |
| 390 | 4-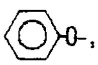O-,2-Cℓ- | H− | −$CH_2$− | i-$C_3H_7$− | $n_D^{23}$ 1.5713 |
| 391 | 2,4-$(CH_3)_2$ | H− | −$CH_2$− | i-$C_3H_7$− | $n_D^{23}$ 1.5688 |
| 392 | 2,4-$(CH_3)_2$ | 5-$CH_3$− | −$CH_2$− | i-$C_3H_7$− | $n_D^{23}$ 1.5643 |
| 393 | 3,4-$(CH_3)_2$ | H− | −$CH_2$− | i-$C_3H_7$− | $n_D^{23}$ 1.5776 |
| 394 | 3,4-$(CH_3)_2$ | 5'-Cℓ− | −$CH_2$− | i-$C_3H_7$− | mp 80.5-82°C |
| 395 | 3,4-$(CH_3)_2$ | 5'-Cℓ− | −$CH_2$− | i-$C_3H_7$− | $n_D^{23}$ 1.5577 |
| 396 | 2-$CH_3$-4-$C_2H_5O$− | H− | −$CH_2$− | i-$C_3H_7$− | $n_D^{23}$ 1.5702 |
| 397 | 2-$CH_3$-4-$CH_3S$− | H− | −$CH_2$− | i-$C_3H_7$− | $n_D^{23}$ 1.5714 |
| 398 | 2-$CH_3$-4-$CH_3S$− | 5'-Cℓ− | −$CH_2$− | i-$C_3H_7$− | $n_D^{23}$ 1.5694 |
| 399 | 2-$CH_3$-4-$CH_3S$− | 5'-$NO_2$-6'-Cℓ− | −$CH_2$− | i-$C_3H_7$− | $n_D^{23}$ 1.5681 |
| 400 | 2-$NO_2$-4-$CH_3$− | H− | −$CH_2$− | i-$C_3H_7$− | $n_D^{23}$ 1.5710 |
| 401 | 2-$NO_2$-4-$CH_3$− | 5'-$NO_2$− | −$CH_2$− | i-$C_3H_7$− | $n_D^{23}$ 1.5715 |
| 402 | 2,4,5-$Cℓ_3$− | H− | −$CH_2$− | i-$C_3H_7$− | $n_D^{23}$ 1.5704 |
| 403 | 2-$CF_3$-4,6-$Cℓ_2$− | H− | −$CH_2$− | i-$C_3H_7$− | $n_D^{23}$ 1.5683 |
| 404 | 2-$CF_3$-4,6-$Cℓ_2$− | H− | −CH−(CH$_3$) | i-$C_3H_7$− | $n_D^{23}$ 1.5681 |
| 405 | 2,6-$F_2$-4-Cℓ− | H− | −$CH_2$− | i-$C_3H_7$− | $n_D^{23}$ 1.5698 |
| 406 | 2,6-$F_2$-4-Cℓ− | 5',6'-$(CH_3)_2$ | −$CH_2$− | i-$C_4H_9$− | $n_D^{23}$ 1.5707 |
| 407 | 2,6-$F_2$-4-Cℓ− | 4'-$CH_3$-6'-Cℓ− | −$CH_2$− | i-$C_3H_7$− | $n_D^{23}$ 1.5725 |
| 408 | 2-$CH_3$-4,6-$Cℓ_2$− | H− | −$CH_2$− | i-$C_3H_7$− | $n_D^{23}$ 1.5741 |
| 409 | 2-F-4-Cℓ-6-$CH_3O$- | H− | −$CH_2$− | i-$C_3H_7$− | $n_D^{23}$ 1.5708 |
| 410 | 2-F-4-Cℓ-6-$CH_3O$- | 5'-Cℓ− | $-(CH_2)_2-$ | sec-$C_4H_9$− | $n_D^{23}$ 1.5710 |

Notes: In Table 1, n- means "normal"; i- means "iso"; and sec- means "secondary". Further, t- means "tertiary". The same apply to Table 2.

The compound of the general formula (I) according to this invention are effective as the herbicidally active ingredient for controlling or combating various sorts of weeds growing in the paddy fields of rice plant and also in the fields of upland crop plants.

Preferred examplary compounds amongst the compounds of the general formula (I) are listed below.

| Compound name (Compound No.) | Structural Formula |
|---|---|
| (1) Benzoxazol-2-yloxyacetic acid N-isobutyl-N-benzyloxyamide (Compound No. 7) | 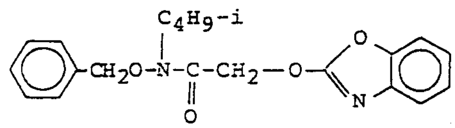 |
| (2) 6-Methylbenzoxazol-2-yloxyacetic acid N-isobutyl-N-benzyloxyamide (Compound No. 66) | 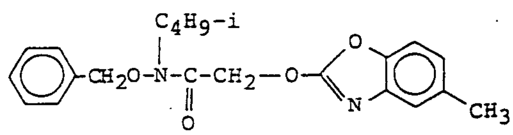 |
| (3) Benzoxazol-2-yloxyacetic acid N-isopropyl-N-benzyloxyamide (Compound No. 5) | 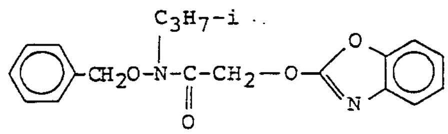 |
| (4) Benzoxazol-2-yloxyacetic acid N-isopropyl-N-o-methylbenzyloxyamide (Compound No. 202) | 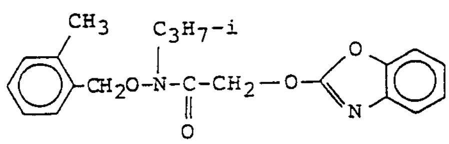 |
| (5) 5-Methylbenzoxazol-2-yloxyacetic acid N-isopropyl-N-benzyloxyamide (Compound No. 53) | 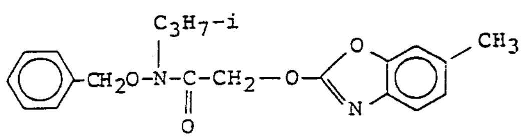 |
| (6) 6-Methylbenzoxazol-2-yloxyacetic acid N-isopropyl-N-benzyloxyamide (Compound No. 65) | 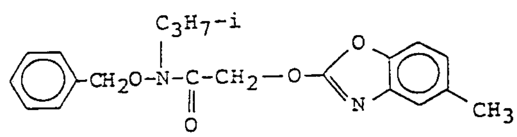 |
| (7) Benzoxazol-2-yloxyacetic acid N-isopropyl-N-m-methylbenzyloxyamide (Compound No. 227) | 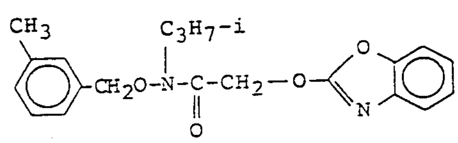 |

Now, a process for the preparation of the compounds of the invention is described below.

The compound of the general formula (I) according to the first aspect of the invention may be prepared by reacting a corresponding 2-chlorobenzoxazole derivative of the general formula (III) with a corresponding hydroxyacetamide derivative of the general formula (II) in the presence of an acid-binding agent in accordance with the following reaction equation:-

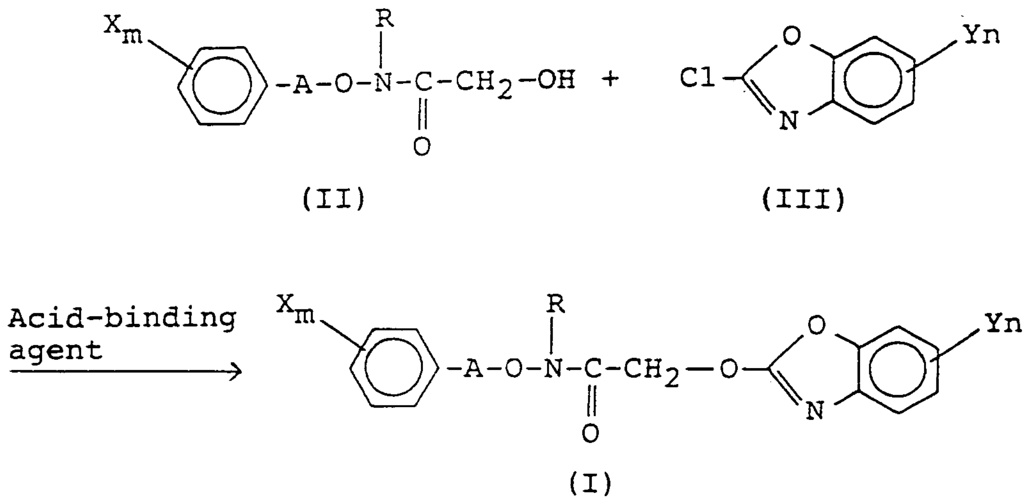


(II) (III)

(I)

wherein X, Y, A, R, m and n have the same meanings as defined above.

The above condensation reaction may usually be conducted in an organic solvent. Usable solvents may include hydrocarbons such as benzene, toluene and hexane; halogenated hydrocarbons such as chloroform and chlorobenzenes; ethers such as ethyl ether, dioxane and tetrahydrofuran; esters such as methyl acetate and ethyl acetate; ketones such as acetone and methyl isobutyl ketone; nitriles such as acetonitrile and propionitrile; amides such as dimethyl-formamide and dimethylacetamide, and dimethylsulfoxide and the like.

As the acid-binding agent, there may be used inorganic base such as sodium hydride,sodium amide, sodium hydroxide or potassium carbonate, or an organic base such as triethylamine or pyridine.

The condensation reaction may proceed at room temperature, but it is also possible to reduce the reaction time by heating the reaction system up to the boiling temperature of the solvent employed. After completion of the reaction, a salt of the acid-binding agent as formed is filtered off from the reaction solution, if it contains such salt. The aimed compound of the formula (I) as produced can then be obtained by distilling off the solvent from the reaction solution. It is also possible to obtain the compound of the formula (I) according to this invention by adding to the reaction solution both water and an organic solvent such as benzene, toluene, tetrahydrofuran or chloroform, separating from the reaction solution an organic extract containing the aimed product and then distilling off the solvents from the organic extract.

Examples for production of certain compounds of this invention according to the above-mentioned process will be described as Examples 1-10 given hereinafter.

Incidentally, the 2-chlorobenzoxazole compounds having the general formula (III) which are the starting material are all known compounds. Whereas, the hydroxyacetamide derivative having the general formula (II) are novel compounds. To prepare the novel compound having the general formula (II), the corresponding aralkyloxyamine and acetoxyacetic acid chloride are reacted with each other to form an acetoxyacetamide derivative, followed by hydrolysis of the ester moiety of the latter derivative. In this manner, the hydroxyacetamide derivatives having the general formula (II) may be obtained as the starting material. Their preparation will be illustrated in Referential Examples 1-6 hereinafter. In addition, some representative examples of the compound of the general formula (II) thus obtained are listed in Table 2.

Example 1

Preparation of benzoxazol-2-yloxyacetic acid N-propargyl-N-benzyloxyamide (Compound No. 10)

21.9 g of hydroxyacetic acid N-propargyl-N-benzyloxyamide were dissolved in 100 ml of tetrahydrofuran, followed by addition of 3.9 g of sodium amide thereto. Then, 15.4 g of 2-chlorobenzoxazole were added further, followed by stirring the resulting mixture at room temperature for 2 hours. After completion of the reaction, toluene and water were added to the resulting reaction solution and an organic layer was then separated therefrom. After washing with water, the organic layer as separated was dried over anhydrous sodium sulfate. The solvent was thereafter distilled off from the organic layer solution under reduced pressure, whereby 31.2 g of the above titled compound were obtained as a pale brown oily substance. This compound crystallized at room temperature. When recrystallized from a mixed solvent of

hexane and ethyl acetate, said compound turned to white crystals. Melting point: 52-53 ° C.

Example 2

Preparation of benzoxazol-2-yloxyacetic acid N-isopropyl-N-para-chlorobenzyloxyamide (Compound No. 126)

A solution of 25.8 g of hydroxyacetic acid N-isopropyl-N-para-chlorobenzyloxyamide in 50 ml of dioxane was added dropwise to a mixture of 100 ml of dioxane and 4.0 g of 60% sodium hydride. After 15.4 g of 2-chlorobenzoxazole were added, the resultant mixture was stirred at room temperature for 3 hours. After completion of the reaction, toluene and water were added to the resulting reaction solution and the resulting mixture was treated in a similar manner to Example 1, thereby obtaining 34.5 g of the above titled compound as pale brown crystals. When recrystallized from a mixed solvent of hexane and acetone, said compound turned to white crystals. Melting point: 87-88 ° C.

Example 3

Preparation of 5-methylbenzoxazol-2-yloxyacetic acid N-isopropyl-N-benzyloxyamide (Compound No. 53)

The procedure of Example 1 was repeated using 22.3 g of hydroxyacetic acid N-isopropyl-N-benzyloxyamide and 16.8 g of 2-chloro-5-methylbenzoxazole for the reaciton. The resulting reaction solution was then after-treated similarly to Example 1 to afford 32.6 g of the above-titled compound as pale brown crystals. When recrystallized from a mixed solvent of hexane and ethyl acetate, said compound turned to white crystals. Melting point: 85-86 ° C.

Example 4

Preapration of 6-methylbenzoxazol-2-yloxyacetic acid N-isopropyl-N-para-chlorobenzyloxyamide (Compound No. 153)

The procedure of Example 2 was repeated using 25.8 g of hydroxyacetic acid N-isopropyl-N-para-chlorobenzyloxyamide and 16.8 g of 2-chloro-6-methylbenzoxazole for the reaciton. The resulting reaction solution was after-treated similarly to Example 2 to afford 35.0 g of the titled compound as pale brown crystals. When recrystallized from a mixed solvent of hexane and acetone, said compound turned to white crystals. Melting point: 87-89 ° C.

Example 5

Preparation of benzoxazol-2-yloxyacetic acid N-isopropyl-N-ortho-methylbenzyloxyamide (Compound No. 202)

The procedure of Example 1 was repeated using 23.7 g of hydroxyacetic acid N-isopropyl-N-ortho-methylbenzyloxyamide and 15.4 g of 2-chlorobenzoxazole for the reaction. The resulting reaction solution was after-treated similarly to Example 1 to yield 32.9 g of the title compound as a pale yellow oily substance. This substance soon crystallized at room temperature. When recrystallized from hexane, said substance turned to white crystals. Melting point: 69.5-71 ° C.

Example 6

Preapration of 5-chlorobenzoxazol-2-yloxyacetic acid N-isopropyl-N-meta-methoxybenzyloxyamide (Compound No. 264)

The procedure of Example 2 was repeated using 25.3 g of hydroxyacetic acid N-isopropyl-N-meta-methoxybenzyloxyamide and 18.8 g of 2,5-dichlorobenzoxazole for the reaciton. The resulting reaction solution was after-treated similarly to Example 2 to afford 33.7 g of the title compound as pale brown crystals. When recrystallized from a mixed solvent of hexane and ethyl acetate, said compound turned to white crystals. Melting point: 70-72 ° C.

Example 7

Preparation of benzoxazol-2-yloxyacetic acid N-isopropyl-N-para-trifluoromethylbenzyloxyamide (Compound No. 297)

The procedure of Example 1 was repeated using 29.1 g of hydroxyacetic acid N-isopropyl-N-para-trifluoromethylbenzyloxyamide and 15.4 g of 2-chlorobenzoxazole for the reaction. The resulting reaction solution was after-treated similarly to Example 1 to give 37.5 g of the title compound as pale brown crystals. When recrystallized from hexane, said compound turned to white crystals. Melting point: 82-84 ° C.

Example 8

Preparation of benzoxazol-2-yloxyacetic acid N-isopropyl-N-(2-chloro-4-phenoxy)benzyloxyamide (Compound No. 390)

The procedure of Example 2 was repeated using 35.0 g of hydroxyacetic acid N-isopropyl-N-(2-chloro-4-phenoxy) benzyloxyamide and 15.4 g of 2-chlorobenzoxazole for the reaciton. The resulting reaction solution was aftertreated similarly to Example 2 to afford 43.4 g of the title compound as an oily, pale yellow substance. Upon its chromatographic purification on a silica gel column by using a mixed solvent of hexane and acetone as the eluent, said substance turned to an oily colorless substance. $n_D^{23}$ = 1.5713.

Example 9

Preapration of 5-fluorobenzoxazol-2-yloxyacetic acid N-isobutyl-N-ortho-methylbenzyloxyamide (Compound No. 207)

The procedure of Example 1 was repeated using 25.1 g of hydroxyacetic acid N-isobutyl-N-ortho-methylbenzyloxyamide and 17.2 g of 2-chloro-5-fluorobenzoxazole for the reaciton. The resulting reaction solution was after-treated similarly to Example 1 to give 35.1 g of the title compound as pale brown crystals. When recrystallized from a mixed solvent of hexane and ethyl acetate, said compound turned to white crystals. Melting point: 68-70 ° C.

Example 10

Preparation of 5-methylbenzoxazol-2-yloxyacetic acid N-isobutyl-N-para-ethoxybenzyloxyamide (Compound No. 277)

The procedure of Example 2 was repeated using 26.7 g of hydroxyacetic acid N-isopropyl-N-paraethoxybenzyloxyamide and 16.8 g of 2-chloro-5-methylbenzoxazole for the reaction. The resulting reaction solution was after-treated similarly to Example 2 to afford 36.6 g of the title compound as pale yellow crystals. When recrystallized from a mixed solvent of cyclohexane and ethyl acetate, said compound turned to white crystals. Melting point: 95-96.5 ° C.

The following Referential Examples illustrate preparation of the starting compound of the formula (II).

Referential Example 1

Preparation of hydroxyacetic acid N-propargyl-N-benzyloxyamide (Compound No. 13' indicated in Table 2)

Under ice cooling, 13.7 g of acetoxyacetic acid chloride were added dropwise to a mixture of 16.1 g of N-propargyl-N-benzyloxyamine, 10.1 g of triethylamine and 200 ml of chloroform. After the dropwise addition, the resultant mixture was stirred at room temperature for 1 hour. Subsequent to the completion of the reaction, water was added to the resulting reacion solution, and the organic layer thus formed was separated from said reaction solution, washed with 1 N hydrochloric acid and then with water, and thereafter dried over anhydrous sodium sulfate. When the solvent was distilled off from the dried solution under reduced pressure, 25.3 g of acetoxyacetic acid N-propargyl-N-benzyloxyamide were obtained as an oily, pale brown substance. This substance in its total quantity was dissolved in 50 ml of methanol and the resulting solution was added dropwise to a solution of 7.3 g of potassium hydroxide in 100 ml of methanol. After the dropwise addition, the resultant mixture was stirred at room temperature for 20 minutes.

After the reaction was completed, toluene and water were added to the resulting reaction solution. An organic layer thus formed was separated from said reaction solution and then dried over anhydrous sodium sulfate. When the solvents were distilled off from the dried solution under reduced pressure, 19.1 g of the title compound were obtained as pale brown oily substance. Upon its chromatographic purification on a silica gel column by using a mixed solvent of toluene and acetone as the eluent, said substance turned to an oily colorless substance. $n_D^{23}$ = 1 5039.

Referential Example 2

Preparation of hydroxyacetic acid N-isopropyl-N-para-chlorobenzyloxyamide (Compound No. 42' indicated in Table 2)

The procedure of Referential Example 1 was repeated using 20.0 g of N-isopropyl-N-para-chlorobenzyloxyamine for the reaction, followed by the after-treatment of the resulting reaction solution similarly to Referential Example 1. 21.9 g of the title compound was afforded as an oily, pale yellow substance. Upon its chromatographic purification on a silica gel column by using a mixed solvent of hexane and ethyl acetate as the eluent, said substance turned to an oily colorless substance. $n_D^{23}$ = 1.5071.

Referential Example 3

Preparation of hydroxyacetic acid N-isopropyl-N-paramethylbenzyloxyamide (Compound No. 74' shown in Table 2)

The procedure of Referential Example 1 was repeated using 17.9 g of N-isopropyl-N-para-methylbenzyloxyamine for the reaction, followed by the after-treatment of the resulting reaction solution similarly to Referential Example 1. 19.1 g of the title compound was afforded as an oily, pale yellow substance. Upon its chromatographic purification on a silica gel column by using a mixed solvent of hexane and ethyl acetate as the eluent, said substance turned to an oily colorless substance. $n_D^{23}$ = 1.5084.

Referential Example 4

Preparation of hydroxyacetic acid N-isopropyl-N-meta-methoxybenzyloxyamide (Compound No. 84' in Table 2)

The procedure of Referential Example 1 was repeated using 19.5 g of N-isopropyl-N-meta-methoxybenzyloxyamine for the reaction, followed by the after-treatment of the resulting reaction solution similarly to Referential Example 1. 20.7 g of the title compound was obtained as an oily, pale yellow substance. Upon its chromatographic purification on a silica gel column by using a mixed solvent of hexane and ethyl acetate as eluent, said substance turned to an oily colorless substance. $n_D^{23}$ = 1.5032.

Referential Example 5

Preparation of hydroxyacetic acid N-isopropyl-N-(2-chloro-4-phenoxy)benzyloxyamide (Compound No. 148' in Table 2)

The procedure of Referential Example 1 was repeated using 29.2 g of N-isopropyl-N-(2-chloro-4-phenoxy)-benzyloxyamine for the reaction, followed by the after-treatment of the resulting reaction solution similarly to Referential Example 1. 29.0 g of the title compound was obtained as an oily, pale yellow substance. Upon its chromatographic purification on a silica gel column by using a mixed solvent of toluene and acetone as an eluent, said substance turned to an oily colorless substance. $n_D^{23}$ = 1.5114.

Referential Example 6

Preparation of hydroxyacetic acid N-isobutyl-N-ortho-methyl-benzyloxyamide (Compound No. 71' in Table 2)

The procedure of Referential Example 1 was repeated using 19.3 g of N-isobutyl-N-ortho-methylbenzyloxyamine for the reaction, followed by the after-treatment of the resulting reaction solution similarly to

Referential Example 1. 21.6 g of the title compound was obtained as an oily, pale yellow substance. Upon its chromatographic purification on a silica gel column by using a mixed solvent of hexane and ethyl acetate as an eluent, said substance turned to an oily colorless substance. $n_D^{23}$ = 1.5116.

The following Table 2 shows a list of some examples of the compound of the formula (II) which have been prepared in the same manner as in the above Referential Examples 1 to 6.

Table 2

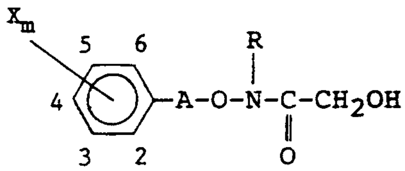

(II)

| Compound No. | $X_m$ | A | R | Physical data (melting point or refractive index) |
|---|---|---|---|---|
| 1′ | H— | $-CH_2-$ | H— | $n_D^{23}$ 1.4937 |
| 2′ | H— | $-CH_2-$ | $CH_3-$ | $n_D^{23}$ 1.4972 |
| 3′ | H— | $-CH_2-$ | $C_2H_5-$ | $n_D^{23}$ 1.4993 |
| 4′ | H— | $-CH_2-$ | $n-C_3H_7-$ | $n_D^{23}$ 1.4948 |
| 5′ | H— | $-CH_2-$ | $i-C_3H_7-$ | $n_D^{23}$ 1.5030 |
| 6′ | H— | $-CH_2-$ | $n-C_4H_9-$ | $n_D^{23}$ 1.4952 |
| 7′ | H— | $-CH_2-$ | $i-C_4H_9-$ | $n_D^{23}$ 1.5008 |
| 8′ | H— | $-CH_2-$ | $sec-C_4H_9-$ | $n_D^{23}$ 1.5052 |
| 9′ | H— | $-CH_2-$ | $i-C_5H_{11}-$ | $n_D^{23}$ 1.5076 |
| 10′ | H— | $-CH_2-$ | $n-C_6H_{13}-$ | $n_D^{23}$ 1.5081 |
| 11′ | H— | $-CH_2-$ | $CH_2=CHCH_2-$ | $n_D^{23}$ 1.5004 |
| 12′ | H— | $-CH_2-$ | $CH_2=C-CH_2-$<br>\|<br>$CH_3$ | $n_D^{23}$ 1.5110 |
| 13′ | H— | $-CH_2-$ | $HC\equiv CCH_2-$ | $n_D^{23}$ 1.5039 |
| 14′ | H— | $-CH_2-$ | 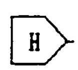 | $n_D^{23}$ 1.5026 |
| 15′ | H— | $-CH_2-$ | 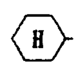 | $n_D^{23}$ 1.5013 |
| 16′ | H— | $-CH_2-$ | $CH_3OCH_2CH_2-$ | $n_D^{23}$ 1.4994 |
| 17′ | H— | $-CH_2-$ | $n-C_4H_9OCH_2CH_2-$ | $n_D^{23}$ 1.4987 |
| 18′ | H— | $-CH_2-$ | $CH_3SCH_2CH_2-$ | $n_D^{23}$ 1.5026 |
| 19′ | H— | $-CH_2-$ | $C_2H_5SCH_2CH_2-$ | $n_D^{23}$ 1.5014 |
| 20′ | H— | $-CH_2-$ | $CH_3OCOCH_2-$ | $n_D^{23}$ 1.4994 |
| 21′ | H— | $-CH_2-$ | $C_2H_5OCOCH_2-$ | $n_D^{23}$ 1.4963 |
| 22′ | H— | $-CH_2-$ | $NCCH_2-$ | $n_D^{23}$ 1.5006 |
| 23′ | H— | $-CH_2-$ | $CH_3COCH_2-$ | $n_D^{23}$ 1.5014 |

Table 2 (Continued-1)

| Compound No. | $X_m$ | A | R | Physical data |
|---|---|---|---|---|
| 24′ | H— | $-CH_2-$ | 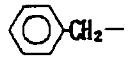 | $n_D^{23}$ 1.5111 |
| 25′ | H— | $-CH-$<br>$CH_3$ | i-$C_3H_7$— | $n_D^{23}$ 1.5121 |
| 26′ | H— | $-(CH_2)_2-$ | i-$C_3H_7$— | $n_D^{23}$ 1.5098 |
| 27′ | H— | $-(CH_2)_2-$ | $CH_3SCH_2CH_2-$ | $n_D^{23}$ 1.5046 |
| 28′ | H— | $-CH-$<br>$C_2H_5$ | $CH_3-$ | $n_D^{23}$ 1.5064 |
| 29′ | H— | $-(CH_2)_3-$ | i-$C_3H_7$— | $n_D^{23}$ 1.5123 |
| 30′ | H— | $-CH-$<br>n-$C_3H_7$— | n-$C_4H_9$— | $n_D^{23}$ 1.5093 |
| 31′ | 2-Cl— | $-CH_2-$ | $C_2H_5-$ | $n_D^{23}$ 1.5046 |
| 32′ | 2-Cl— | $-CH_2-$ | i-$C_3H_7$— | $n_D^{23}$ 1.4920 |
| 33′ | 2-Cl— | $-CH_2-$ | $CH_2{=}CHCH_2-$ | $n_D^{23}$ 1.4998 |
| 34′ | 2-Cl— | $-CH_2-$ | $HC{\equiv}C\text{-}CH-$<br>$CH_3$ | $n_D^{23}$ 1.5006 |
| 35′ | 2-Cl— | $-CH_2-$ | $CH_3C{\equiv}CCH_2-$ | $n_D^{23}$ 1.5041 |
| 36′ | 2-Cl— | $-CH_2-$ | n-$C_3H_7OCH_2CH_2-$ | $n_D^{23}$ 1.5039 |
| 37′ | 2-Cl— | $-CH-$<br>$CH_3$ | $C_2H_5-$ | $n_D^{23}$ 1.5041 |
| 38′ | 2-Cl— | $-CH-$<br>$CH_3$ | 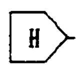 | $n_D^{23}$ 1.5062 |
| 39′ | 3-Cl— | $-CH_2-$ | i-$C_3H_7$— | $n_D^{23}$ 1.5070 |
| 40′ | 3-Cl— | $-(CH_2)_2-$ | i-$C_3H_7$— | $n_D^{23}$ 1.5038 |
| 41′ | 4-Cl— | $-CH_2-$ | $CH_3-$ | $n_D^{23}$ 1.5062 |
| 42′ | 4-Cl— | $-CH_2-$ | i-$C_3H_7$— | $n_D^{23}$ 1.5071 |
| 43′ | 4-Cl— | $-CH_2-$ | i-$C_4H_9$— | $n_D^{23}$ 1.5041 |
| 44′ | 4-Cl— | $-CH_2-$ | sec-$C_4H_9$— | $n_D^{23}$ 1.5066 |
| 45′ | 4-Cl— | $-CH_2-$ | $CH_2{=}CHCH_2-$ | $n_D^{23}$ 1.5079 |

Table 2 (Continued-2)

| Compound No. | $X_m$ | A | R | Physical data |
|---|---|---|---|---|
| 46′ | 4-Cl— | $—CH_2—$ | $HC≡CCH_2—$ | mp 69-70℃ |
| 47′ | 4-Cl— | $—CH_2—$ | 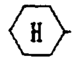 | $n_D^{23}$ 1.5028 |
| 48′ | 4-Cl— | $—CH_2—$ | $CH_3SCH(CH_3)-CH_2—$ | $n_D^{23}$ 1.5036 |
| 49′ | 4-Cl— | $—CH_2—$ | $NCCH_2CH_2—$ | $n_D^{23}$ 1.5040 |
| 50′ | 4-Cl— | $—CH_2—$ | 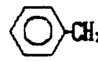 $-CH_2CH_2—$ | $n_D^{23}$ 1.4998 |
| 51′ | 4-Cl— | $—CH(CH_3)—$ | $i-C_3H_7—$ | $n_D^{23}$ 1.5048 |
| 52′ | 4-Cl— | $-(CH_2)_3-$ | $i-C_3H_7—$ | $n_D^{23}$ 1.5072 |
| 53′ | 2-F— | $—CH_2—$ | $i-C_3H_7—$ | $n_D^{23}$ 1.5061 |
| 54′ | 2-F— | $-(CH_2)_2-$ | $n-C_5H_{11}—$ | $n_D^{23}$ 1.5042 |
| 55′ | 3-F— | $—CH_2—$ | $i-C_3H_7—$ | $n_D^{23}$ 1.5073 |
| 56′ | 4-F— | $—CH_2—$ | H— | $n_D^{23}$ 1.5043 |
| 57′ | 4-F— | $—CH_2—$ | $i-C_3H_7—$ | $n_D^{23}$ 1.5014 |
| 58′ | 4-F— | $—CH_2—$ | $sec-C_4H_9—$ | $n_D^{23}$ 1.5006 |
| 59′ | 4-F— | $—CH_2—$ | $HC≡CCH_2—$ | $n_D^{23}$ 1.5000 |
| 60′ | 4-F— | $—CH_2—$ | $CH_3OCH_2CH_2—$ | $n_D^{23}$ 1.5006 |
| 61′ | 4-F— | $—CH(CH_3)—$ | $i-C_3H_7—$ | $n_D^{23}$ 1.5041 |
| 62′ | 4-F— | $-(CH_2)_2-$ | $n-C_3H_7—$ | $n_D^{23}$ 1.5030 |
| 63′ | 4-F— | $-(CH_2)_3-$ | $CH_3—$ | $n_D^{23}$ 1.5023 |
| 64′ | 2-Br— | $—CH_2—$ | $i-C_3H_7—$ | $n_D^{23}$ 1.5021 |
| 65′ | 3-Br— | $—CH_2—$ | $i-C_3H_7—$ | $n_D^{23}$ 1.5081 |
| 66′ | 4-Br— | $—CH_2—$ | $i-C_3H_7—$ | $n_D^{23}$ 1.5029 |
| 67′ | 4-Br— | $—CH_2—$ | $sec-C_4H_9—$ | $n_D^{23}$ 1.5035 |
| 68′ | 4-Br— | $—CH_2—$ | 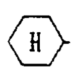 | $n_D^{23}$ 1.5043 |

Table 2 (Continued-3)

| Compound No. | $X_m$ | A | R | Physical data |
|---|---|---|---|---|
| 69' | 2-$CH_3$— | —$CH_2$— | $C_2H_5$— | $n_D^{23}$ 1.5048 |
| 70' | 2-$CH_3$— | —$CH_2$— | i-$C_3H_7$— | $n_D^{23}$ 1.5068 |
| 71' | 2-$CH_3$— | —$CH_2$— | i-$C_4H_9$— | $n_D^{23}$ 1.5116 |
| 72' | 3-$CH_3$— | —$CH_2$— | i-$C_3H_7$— | $n_D^{23}$ 1.5035 |
| 73' | 3-$CH_3$— | —$CH_2$— | $CH_2$=$CHCH_2$— | $n_D^{23}$ 1.5066 |
| 74' | 4-$CH_3$— | —$CH_2$— | i-$C_3H_7$— | $n_D^{23}$ 1.5084 |
| 75' | 4-$CH_3$— | —$CH_2$— | i-$C_4H_9$— | $n_D^{23}$ 1.5095 |
| 76' | 4-$CH_3$— | $-(CH_2)_2-$ | $C_2H_5$— | $n_D^{23}$ 1.5074 |
| 77' | 2-$C_2H_5$— | —$CH_2$— | i-$C_3H_7$— | $n_D^{23}$ 1.5110 |
| 78' | 4-$C_2H_5$— | —$CH_2$— | i-$C_3H_7$— | $n_D^{23}$ 1.5108 |
| 79' | 4-n-$C_3H_7$— | —$CH_2$— | i-$C_3H_7$— | $n_D^{23}$ 1.5073 |
| 80' | 4-i-$C_3H_7$— | —$CH_2$— | i-$C_3H_7$— | $n_D^{23}$ 1.5110 |
| 81' | 4-t-$C_4H_9$— | —$CH_2$— | i-$C_3H_7$— | $n_D^{23}$ 1.5024 |
| 82' | 4-t-$C_4H_9$— | $-(CH_2)_3-$ | i-$C_3H_7$— | $n_D^{23}$ 1.4996 |
| 83' | 2-$CH_3O$— | —$CH_2$— | i-$C_3H_7$— | $n_D^{23}$ 1.4987 |
| 84' | 3-$CH_3O$— | —$CH_2$— | i-$C_3H_7$— | $n_D^{23}$ 1.5032 |
| 85' | 4-$CH_3O$— | —$CH_2$— | i-$C_3H_7$— | $n_D^{23}$ 1.5067 |
| 86' | 2-$C_2H_5O$— | —$CH_2$— | i-$C_3H_7$— | $n_D^{23}$ 1.5044 |
| 87' | 4-$C_2H_5O$— | —$CH_2$— | i-$C_3H_7$— | $n_D^{23}$ 1.5025 |
| 88' | 4-n-$C_3H_7O$— | —$CH_2$— | i-$C_3H_7$— | $n_D^{23}$ 1.4983 |
| 89' | 2-i-$C_3H_7O$— | —$CH_2$— | i-$C_3H_7$— | $n_D^{23}$ 1.5128 |
| 90' | 2-$CF_3$— | —$CH_2$— | i-$C_3H_7$— | $n_D^{23}$ 1.5021 |
| 91' | 3-$CF_3$— | —$CH_2$— | i-$C_3H_7$— | $n_D^{23}$ 1.5043 |
| 92' | 4-$CF_3$— | —$CH_2$— | i-$C_3H_7$— | $n_D^{23}$ 1.5049 |
| 93' | 4-$CF_3$— | —CH($CH_3$)— | $C_2H_5$— | $n_D^{23}$ 1.5021 |
| 94' | 2-$CHF_2O$— | —$CH_2$— | i-$C_3H_7$— | $n_D^{23}$ 1.5036 |
| 95' | 3-$CHF_2O$— | —$CH_2$— | i-$C_3H_7$— | $n_D^{23}$ 1.5015 |
| 96' | 4-$CHF_2O$— | —$CH_2$— | i-$C_3H_7$— | $n_D^{23}$ 1.5026 |

Table 2 (Continued-4)

| Compound No. | $X_m$ | A | R | Physical data |
|---|---|---|---|---|
| 97′ | 4-$CHF_2O-$ | $-(CH_2)_2-$ | $HC\equiv CCH-$<br>$\vert$<br>$CH_3$ | $n_D^{23}$ 1.5046 |
| 98′ | 4-$CF_3O-$ | $-CH_2-$ | i-$C_3H_7-$ | $n_D^{23}$ 1.5015 |
| 99′ | 2-$CH_3S-$ | $-CH_2-$ | i-$C_3H_7-$ | $n_D^{23}$ 1.5026 |
| 100′ | 3-$CH_3S-$ | $-CH_2-$ | i-$C_3H_7-$ | $n_D^{23}$ 1.5037 |
| 101′ | 4-$CH_3S-$ | $-CH_2-$ | i-$C_3H_7-$ | $n_D^{23}$ 1.5042 |
| 102′ | 4-$CH_3S-$ | $-(CH_2)_2-$ | i-$C_3H_7-$ | $n_D^{23}$ 1.5026 |
| 103′ | 4-$C_2H_5S-$ | $-CH_2-$ | i-$C_3H_7-$ | $n_D^{23}$ 1.5015 |
| 104′ | 4-n-$C_3H_7S-$ | $-CH_2-$ | i-$C_3H_7-$ | $n_D^{23}$ 1.4975 |
| 105′ | 4-$CH_3SO-$ | $-CH_2-$ | i-$C_3H_7-$ | $n_D^{23}$ 1.4995 |
| 106′ | 4-$CH_3SO_2-$ | $-CH_2-$ | i-$C_3H_7-$ | $n_D^{23}$ 1.5021 |
| 107′ | 2-$CH_3CO-$ | $-CH_2-$ | i-$C_3H_7-$ | $n_D^{23}$ 1.5036 |
| 108′ | 3-$CH_3CO-$ | $-CH_2-$ | i-$C_3H_7-$ | $n_D^{23}$ 1.5068 |
| 109′ | 4-$CH_3CO-$ | $-CH_2-$ | i-$C_3H_7-$ | $n_D^{23}$ 1.5040 |
| 110′ | 4-$NO_2-$ | $-CH_2-$ | i-$C_3H_7-$ | $n_D^{23}$ 1.4997 |
| 111′ | 4-CN— | $-CH_2-$ | i-$C_3H_7-$ | $n_D^{23}$ 1.5028 |
| 112′ | 4-$C_2H_5OCO-$ | $-CH_2-$ | i-$C_3H_7-$ | $n_D^{23}$ 1.5035 |
| 113′ | 2- 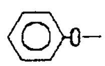 O— | $-CH_2-$ | $CH_3-$ | $n_D^{23}$ 1.5068 |
| 114′ | 2- 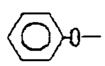 O— | $-CH_2-$ | i-$C_3H_7-$ | $n_D^{23}$ 1.5045 |
| 115′ | 3- 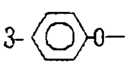 O— | $-CH_2-$ | $CH_3-$ | $n_D^{23}$ 1.5022 |
| 116′ | 3- 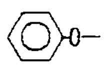 O— | $-CH_2-$ | $C_2H_5-$ | $n_D^{23}$ 1.4998 |
| 117′ | 3- 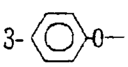 O— | $-CH_2-$ | n-$C_3H_7-$ | $n_D^{23}$ 1.5011 |
| 118′ | 3- 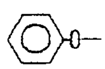 O— | $-CH_2-$ | i-$C_3H_7-$ | $n_D^{23}$ 1.5018 |
| 119′ | 4- 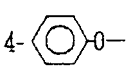 O— | $-CH_2-$ | $CH_3-$ | $n_D^{23}$ 1.5024 |

Table 2 (Continued-5)

| Compound No. | $X_m$ | A | R | Physical data |
|---|---|---|---|---|
| 120′ | 4- 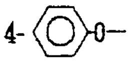 | $-CH_2-$ | $i-C_3H_7-$ | $n_D^{23}$ 1.4978 |
| 121′ | 4- 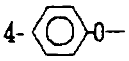 | $-(CH_2)_3-$ | $C_2H_5-$ | $n_D^{23}$ 1.5023 |
| 122′ | 2- 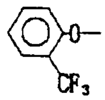 | $-CH_2-$ | $i-C_3H_7-$ | $n_D^{23}$ 1.5046 |
| 123′ | 3-Cl 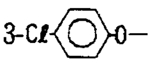 | $-CH_2-$ | $i-C_3H_7-$ | $n_D^{23}$ 1.5021 |
| 124′ | 3-F 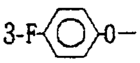 | $-CH(CH_3)-$ | $CH_3-$ | $n_D^{23}$ 1.5067 |
| 125′ | 3- 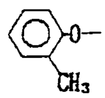 | $-CH_2-$ | $i-C_3H_7-$ | $n_D^{23}$ 1.5001 |
| 126′ | 4-Cl 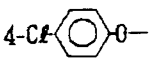 | $-CH_2-$ | $i-C_3H_7-$ | $n_D^{23}$ 1.5036 |
| 127′ | 4-F 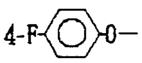 | $-CH(CH_3)-$ | $CH_3-$ | $n_D^{23}$ 1.4978 |
| 128′ | $4-CH_3$ 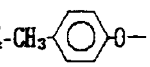 | $-CH_2-$ | $i-C_3H_7-$ | $n_D^{23}$ 1.4982 |
| 129′ | $4-t-C_4H_9$ 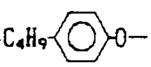 | $-CH(CH_3)-$ | $CH_3-$ | $n_D^{23}$ 1.5006 |
| 130′ | $4-CH_3O$ 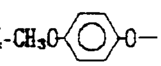 | $-CH_2-$ | $i-C_3H_7-$ | $n_D^{23}$ 1.5072 |
| 131′ | $2,4-Cl_2-$ | $-CH_2-$ | $i-C_3H_7-$ | $n_D^{23}$ 1.5069 |
| 132′ | $2,5-Cl_2-$ | $-CH_2-$ | $i-C_3H_7-$ | $n_D^{23}$ 1.5046 |
| 133′ | $3,4-Cl_2-$ | $-CH_2-$ | $i-C_3H_7-$ | $n_D^{23}$ 1.5077 |
| 134′ | 2-Cl-4-F− | $-CH_2-$ | $i-C_3H_7-$ | $n_D^{23}$ 1.5087 |
| 135′ | 2-Cl-4-F− | $-CH(C_2H_5)-$ | $n-C_5H_{11}-$ | $n_D^{23}$ 1.5015 |
| 136′ | 2-Cl-4-Br− | $-CH_2-$ | $i-C_3H_7-$ | $n_D^{23}$ 1.5024 |
| 137′ | 3-Cl-4-Br− | $-CH(CH_3)-$ | $C_2H_5-$ | $n_D^{23}$ 1.5482 |
| 138′ | 2-Br-4-Cl− | $-CH_2-$ | $i-C_3H_7-$ | $n_D^{23}$ 1.4993 |

Table 2 (Continued-6)

| Compound No. | $X_m$ | A | R | Physical data |
|---|---|---|---|---|
| 139′ | 2-Cl-4-$CH_3$− | $-CH_2-$ | i-$C_3H_7$− | $n_D^{23}$ 1.4968 |
| 140′ | 2-Cl-4-$CH_3$− | $-(CH_2)_2-$ | n-$C_3H_7$− | $n_D^{23}$ 1.5026 |
| 141′ | 2-$CH_3$-4-Cl− | $-CH_2-$ | i-$C_3H_7$− | $n_D^{23}$ 1.5143 |
| 142′ | 2-$CH_3O$-4-Cl− | $-CH_2-$ | i-$C_3H_7$− | $n_D^{23}$ 1.4987 |
| 143′ | 2-$CF_3$-4-Cl− | $-CH_2-$ | i-$C_3H_7$− | $n_D^{23}$ 1.4962 |
| 144′ | 2-Cl-4-$CHF_2O$− | $-CH_2-$ | i-$C_3H_7$− | $n_D^{23}$ 1.4975 |
| 145′ | 2-Cl-4-$CH_3SO_2$− | $-CH_2-$ | i-$C_3H_7$− | $n_D^{23}$ 1.5006 |
| 146′ | 2-Cl-4-$NO_2$− | $-CH_2-$ | i-$C_3H_7$− | $n_D^{23}$ 1.5014 |
| 147′ | 2-Cl-4-$NO_2$− | $-CH(CH_3)-$ | $CH_3OCH_2CH_2$− | $n_D^{23}$ 1.5076 |
| 148′ | 2-Cl-4-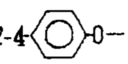O− | $-CH_2-$ | i-$C_3H_7$− | $n_D^{23}$ 1.5114 |
| 149′ | 2,4-$(CH_3)_2$ | $-CH_2-$ | i-$C_3H_7$− | $n_D^{23}$ 1.5043 |
| 150′ | 3,4-$(CH_3)_2$ | $-CH_2-$ | i-$C_3H_7$− | $n_D^{23}$ 1.5042 |
| 151′ | 2-$CH_3$-4-$C_2H_5O$− | $-CH_2-$ | i-$C_3H_7$− | $n_D^{23}$ 1.5068 |
| 152′ | 2-$CH_3$-4-$CH_3S$− | $-CH_2-$ | i-$C_3H_7$− | $n_D^{23}$ 1.4976 |
| 153′ | 2-$NO_2$-4-$CH_3$− | $-CH_2-$ | i-$C_3H_7$− | $n_D^{23}$ 1.4992 |
| 154′ | 2,4,5-$Cl_3$− | $-CH_2-$ | i-$C_3H_7$− | $n_D^{23}$ 1.5073 |
| 155′ | 2-$CF_3$-4,6-$Cl_2$− | $-CH_2-$ | i-$C_3H_7$− | $n_D^{23}$ 1.5114 |
| 156′ | 2-$CF_3$-4,6-$Cl_2$− | $-CH(CH_3)-$ | i-$C_3H_7$− | $n_D^{23}$ 1.5106 |
| 157′ | 2,6-$F_2$-4-Cl− | $-CH_2-$ | i-$C_3H_7$− | $n_D^{23}$ 1.5043 |
| 158′ | 2,6-$F_2$-4-Cl− | $-CH_2-$ | i-$C_4H_9$− | $n_D^{23}$ 1.4987 |
| 159′ | 2-$CH_3$-4,6-$Cl_2$− | $-CH_2-$ | i-$C_3H_7$− | $n_D^{23}$ 1.4886 |
| 160′ | 2-F-4-Cl-6-$CH_3O$− | $-CH_2-$ | i-$C_3H_7$− | $n_D^{23}$ 1.4892 |
| 161′ | 2-F-4-Cl-6-$CH_3O$− | $-(CH_2)_2-$ | sec-$C_4H_9$− | $n_D^{23}$ 1.5006 |

A description will next be made of some methods for formulating the herbicidal compositions containing the compound of the general formula (I) as the active ingredient.

The herbicidal composition according to the second aspect of this invention can be formulated in a suitable form, such as an emulsifiable concentrate, wettable powder, liquor, flowable (sol) preparation, dust, driftless (DL) preparation, granules, fine granules or tablets, by mixing the aralkyloxyamine derivative of the general formula (I) with a liquid or solid carrier or vehicle in a manner known per se in the art. Any solid or liquid carrier or vehicle which may be incorporated into the herbicidal composition as formulated may be conventional one, either solid or liquid, as long as it is routinely employed in the formulations of agricultural and horticultural utilities, and no particular limitation is imposed on the sort of usable carrier or vehicle. Examples of the carrier may include mineral powders such as kaolin, bentonite, clay, montmorillonite, talc, diatomaceous earth, mica, vermiculite, gypsum, calcium carbonate, apatite, white carbon, slaked lime, silica

sand, ammonium sulfate, urea and the like; vegetable flours or meals such as soybean meal, wheat flour, wood meal, tobacco meal, starch, crystalline cellulose and the like; high molecular compounds such as petroleum resins, polyvinyl chloride, ketone resins, dammar gum and the like; alumina, silicates, polysaccharides, highly-dispersible silicic acid, waxes, and so on. On the other hand, liquid carriers may include water, alcohols such as methyl alcohol, ethyl alcohol, n-propyl alcohol, isopropyl alcohol, butanol, ethylene glycol, benzyl alcohol and the like; aromatic hydrocarbons such as toluene, benzene, xylene, ethylbenzene, methylnaphtalene and the like; halogenated hydrocarbons such as chloroform, carbon tetrachloride, dichloromethane, chloroethylene, monochlorobenzene, trichlorofluoromethane, dichlorofluoromethane and the like; ethers such as ethyl ether, ethylene oxide, dioxane, tetrahydrofuran and the like; ketones such as acetone, methyl ethyl ketone, cyclohexanone, methyl isobutyl ketone, isophorone and the like; esters such as ethyl acetate, butyl acetate, ethylene glycol acetate, amyl acetate and the like; acid amides such as dimethylformamide, dimethylacetamide and the like; nitriles such as acetonitrile, propionitrile, acrylonitrile and the like; sulfoxides such as dimethyl sulfoxide and the like; alcohol ethers such as ethylene glycol monomethyl ether, ethylene glycol monoethyl ether and the like; aliphatic and alicyclic hydrocarbons such as n-hexane, cyclohexane and the like; industrial gasoline such as petroleum ether, solvent naphtha and the like; petroleum fractions such as paraffins, kerosine, gas oil, and the like.

Upon formulating such preparations in the form of emulsifiable concentrate, wettable powders and flowable preparations, various surfactants (or emulsifiers) may be used for the purposes of emulsification, dispersion, solubilization, wetting, foaming, lubrication, spreading or the like. Such usable surfactants may include nonionic surfactants (polyoxyethylene alkyl ethers, polyoxyethylene alkyl esters, polyoxyethylene sorbitanalkyl esters, etc.); anionic surfactants (alkyl benzensulfonates, alkyl sulfosuccinates, alkyl sulfates, polyoxyethylene alkyl sulfates, aryl sulfonates, etc.); cationic surfactants [alkyl amines (e.g. lauryl amine, stearyl trimethyl ammonium chloride, alkyl dimethyl benzyl ammonium chlorides, etc.), polyoxyethylene alkyl amines]; amphoteric surfactants [carboxylic acids (e.g. betaine type ones), sulfate esters and salts]; and the like. Needless to say, usable surfactants are not limited to the above exemplified ones only.

In addition, various auxiliary agents can also be used, such as polyvinyl alcohol (PVA), carboxymethylcellulose (CMC), gum arabic, polyvinyl acetate, gelatin, sodium alginate and tragacanth gum.

Upon application of the compound of the formula (I) according to this invention as the herbicide, such compound may be employed in combination with other known herbicides, insecticides, plant growth regulators, or the like. Such combined use can enlarge the applicability of the herbicide (in respect of applicable weeds, methods for application or time stage of application, etc.). In some instances, synergistic effects may be expected by mutual co-operative effects of the individual active ingredients.

Examples of known herbicidal compounds which can exhibit such synergistic effects will next be described, for sake of reference. Thus, granular preparations comprising certain representative compound(s) of this invention and granules containing certain known herbicidal compounds, which had been prepared in accordance with the procedure of Example 11 given hereinafter, were mixed together in various combinations and then the mixtures were tested at various concentrations of the active ingredients in accordance with the procedure of Test Example 1 to estimate what herbicidal effects are achievable against various weeds growing in the paddy field of rice plant. As a result, it has been found that such combinations of the herbicidal compounds from which the synergistic effects are obtainable against at least one species of weed are such mixtures of one or more compounds of this invention with one or more of the following known herbicidal Compounds listed under (a) to (z) below:

(a) methyl α-(4,6-dimethoxypyrimidin-2-ylcarbamoyl-sulfamoyl)-o-toluate;
(b) ethyl 5-[3-(4,6-dimethoxypyrimidin-2-yl)-ureido-sulfonyl]-1-methylpyrazole-4-carboxylate;
(c) 1-[2-(2-methoxyethoxy)]-phenylsolfonyl-3-(4,6-dimethoxy-1,3,5-triazin-2-yl)urea;
(d) 4-(2,4-dichlorobenzoyl)-1,3-dimethyl-5-pyrazolyl-p-toluenesulfonate;
(e) 4-(2,4-dichlorobenzoyl)-1,3-dimethyl-5-phenacyl-oxypyrazole;
(f) 2-[4-(2,4-dichloro-m-toluoyl)-1,3-dimethylpyrazol-5-yloxy]-4'-methylacetophenone;
(g) α-(2-naphthoxy)propionanilide,
(h) 2-(2,4-dichloro-m-tolyloxy)propionanilide;
(i) ethyl 4-chloro-o-tolyloxythioacetate;
(j) 3-isopropyl-2,1,3-benzo-thiadiazinon-(4)-2,2-dioxide;
(k) S-ethyl-hexahydro-1H-azepin-1-carbothioate;
(l) S-(4-chlorobenzyl)-N,N-diethylthiocarbamate;
(m) S-benzyl-N-(1,2-dimethylpropyl)-N-ethylthiocarbamate
(n) S-α,α-dimethylbenzylpiperidine-1-carbothioate;
(o) 2-chloro-2',6'-diethyl-N-(2-propoxyethyl)acetanilide;
(p) 2-chloro-2',6'-diethyl-N-butoxymethylacetanilide;

(q) N-methyl-2-(benzthiazol-2-yl)oxyacetanilide;
(r) S,S-diemthyl 2-(difluoromethyl)-4-(2-methylpropyl)-6-(trifluoromethyl)-3,5-pyridinecarbothioate;
(s) 3,7-dichloro-8-quinolinecarboxylic acid;
(t) 2-bromo-N-(α,α-dimethylbenzyl)-3,3-dimethylbutyl;
(u) 1-(α,α-dimethylbenzyl)-3-p-tolylurea;
(v) 3,4-dichloropropionanilide;
(w) dithiophosphoric acid S-(2-methylpiperidino-carbonylmethyl)-O,O-diisopropyl ester;
(x) 5-t-butyl-3-(2,4-dichloro-5-isopropoxyphenyl)-1,3,4,-oxadiazolidin-2-one;
(y) 2-methylthio-4,6-bis-ethylamino-S-triazine; and
(z) N-(1,2-dimethylpropyl)-N'-ethyl-6-(1,2-dimethylpropylamino)-S-triazine.

Examples of some formulations of the herbicidal composition which comprises the combination of the compound of the formula (I) according to this invention with the known herbicidal compounds capable of exhibiting the above-mentioned synergistic effects will be illustrated in Referential Formulation Examples 1-6.

In the present invention, various formulations described above can be formulated so that the compound of the invention of the general formula (I) are contained in the formulation in a range of 0.001% to 95% (wt.%; all designations of "%" will hereinafter given in wt.%), preferably in a range of 0.01% to 90%. For example, dusts, DL dusts and fine dusts can generally contain the compound of the formula (I) in a range of 0.01% to 10%, while wettable powders, smulsifiable concentrate and liquors can generally contain the compound (I) in a range of 1% to 75%.

Preparations which are formulated as described above, for example, the granular preparations can be applied as such to the surface of soil or into soil or water at a rate of application in a range of about 0.3 - 300 g of the active compound of the general formula (I) of this invention as an active ingredient per 10 ares. In the case of wettable powders, emulsifiable concentrate and sols, they can be applied at a rate of application in a range of about 0.3 - 300 g of the active ingredient per 10 ares, after diluting them with water or an appropriate solvent.

Methods for formulating the compound of the invention of the general formula (I) into herbicidal compositions will herinafter be described with reference to Examples 11 - 24. It should however be borne in mind that this invention is not limited to these Examples but the compounds of this invention can be formulated by mixing various other additives at desired ratios or by mixing at desired ratios the other herbicidal compounds such as those described above.

In the following Examples 11 - 24, Compound Nos. are as shown in Table 1, and all designations of "part" or "parts" are given in part or parts by weight.

Example 11 (Granules)

Granules containing 1% of an active ingredient were prepared by mixing together 15 parts of water with 1 part of Compound No. 126, 1 part of lauryl sulfate, 1 part of calcium lignin sulfonate, 30 parts of bentonite and 67 parts of white clay, kneading the resultant mixture in a kneader, forming the thus-kneaded mass into granules by a granulator and then drying them in a fluidizing drier.

Example 12 (Granules)

Granules containing 1% of an active ingredient were prepared by using 1 part of Compound No. 232 and formulating it in a similar manner to Example 11.

Example 13 (Granules)

Granules containing 1% of an active ingredient were prepared by using 1 part of Compound No. 202 and formulating it in a similar manner to Example 11.

Example 14 (Granules)

Granules containing 1% of an active ingredient were prepared by using 1 part of Compound No. 207 and formulating it in a similar manner to Example 11.

Example 15 (Wettable powder)

A wettable powder containing 15% of an active ingredient was prepared by uniformly mixing together 15 parts of Compound No. 135, 15 parts of white carbon, 3 parts of calcium lignin sulfonate, 2 parts of polyoxyethylene nonylphenyl ether, 5 parts of diatomaceous earth and 60 parts of clay in a grinding mixer.

Example 16 (Wettable powder)

A wettable powder containing 15% of an active ingredient was prepared by using 15 parts of Compound No. 53 and formulating it in a similar manner to Example 15.

Example 17 (Wettable powder)

A wettable powder containing 15% of an active ingredient was prepared by using 15 parts of Compound No. 297 and formulating it in a similar manner to Example 15.

Example 18 (Wettable powder)

A wettable powder containing 15% of an active ingredient was prepared by using 15 parts of Compound No. 277 and formulating it in a similar manner to Example 15.

Example 19 (Emulsifiable Concentrate)

An emulsifiable concentrate containing 20% of an active ingredient was prepared by mixing together 20 parts of Compound No. 10, 20 parts of "Solpole 700H" (trade name for an emulsifier produced by Toho Chemical Industry Co., Ltd.) and 60 parts of xylene.

Example 20 (Emulsifiable Concentrate)

An emulsifiable concentrate containing 20% of an active ingredient was prepared by using 20 parts of Compound No. 65 and formulating it in a similar manner to Example 19.

Example 21 (Emulsifiable Concentrate)

An emulsifiable concentrate containing 20% of an active ingredient was prepared by using 20 parts of Compound No. 390 and formulating it in a similar manner to Example 19.

Example 22 (Dust)

A dust containing 0.5% of an active ingredient was prepared by uniformly mixing together and grinding 0.5 part of Compound No. 23, 0.5 part of fine anhydrous silica powder, 0.5 part of calcium stearate, 50 parts of clay and 48.5 parts of talc.

Example 23 (Dust)

A dust containing 0.5% of an active ingredient was prepared by using 0.5 part of Compound No. 153 and formulating it in a similar manner to Example 22.

Example 24 (Dust)

A dust containing 0.5% of an active ingredient was prepared by using 0.5 part of Compound No. 264 and formulating it in a similar manner to Example 22.

Referential Formulation Example 1

Mixed granular preparations comprising combinations of compounds of this invention with the known Compound (a)

Granular formulations containing 2% of Compound Nos. 10, 53, 126, 153, 202, 207, 264, 277, 297 and 390 according to this invention, respectively, in combination with 0.17% of the known Compound (a) were

each prepared by using 2 parts of the compound of this invention, 0.17 part of the known Compound (a), 1 part of lauryl sulfate, 1 part of calcium lignin sulfonate, 30 parts of bentonite and 65.83 parts of white clay, mixing them and formulating them in a manner similar to Example 11.

Referential Formulation Example 2

Mixed granular preparations comprising combinations of compounds of this invention with the known Compound (b)

Granular formulations containing 1.5% of Compound Nos. 10, 53, 126, 153, 202, 207, 264, 277, 297 and 390 of this invention, respectively, in combination with 0.07% of the known Compound (b) were each prepared by using 1.5 parts of the compound of this invention, 0.07 part of the known Compound (b), 1 part of lauryl sulfate, 1 part of calcium lignin sulfonate, 30 parts of bentonite and 66.43 parts of white clay, mixing them and formulating them in a manner similar to Example 11.

Referential Formulation Example 3

Mixed granular preparations comprising combinations of compounds of this invention with the known Compound (d)

Granular formulations containing 2.5% of Compound Nos. 10, 53, 126, 153, 202, 207, 264, 277, 297 and 390 of this invention, respectively in combination with 7% of the known Compound (d) were each prepared by using 2.5 parts of the compound of this invention, 7 parts of the known Compound (d), 1 part of lauryl sulfate, 1 part of calcium lignin sulfonate, 30 parts of bentonite and 58.5 parts of white clay, mixing them and formulating them in a manner similar to Example 11.

Referential Formulation Example 4

Mixed granular preparations comprising combinations of compounds of this invention with the known Compounds (i) and (y)

Granular formulations containing 2% of Compound Nos. 10, 53, 126, 153, 202, 207, 264, 277, 297 and 390 of this invention, respectively in combination with 0.7% of the known Compound (i) and 1.5% of the known Compound (y) were each prepared by using 2 parts of the compound of this invention, 0.7 part of the known Compound (i), 1.5 parts of the known Compound (y), 1 part of lauryl sulfate, 1 part of calcium lignin sulfonate, 30 parts of bentonite and 63.8 parts of white clay, mixing and formulating them in a manner similar to Example 11.

Referential Formulation Example 5

Mixed granular preparations comprising combinations of compounds of this invention with the known Compounds (a) and (r)

Granular formulations containing 1.5% of Compound Nos. 10, 53, 126, 153, 202, 207, 264, 277, 297 and 390 of this invention, respectively in combination with 0.17% of the known Compound (a) and 0.3% of the known Compound (r) were each prepared by using 1.5 parts of the compound of this invention, 0.17 part of the known Compound (a), 0.3 part of the known Compound (r), 1 part of lauryl sulfate, 1 part of calcium lignin sulfonate, 30 parts of bentonite and 66.03 parts of white clay, mixing and formulating them in a manner similar to Example 11.

Referential Formulation Example 6

Mixed granular preaprations comprising combinations of compounds of the invention with the known Compounds (r) and (s)

Granular formulations containing 1.5% of Compound Nos. 10, 53, 126, 153, 202, 207, 264, 277, 297 and 390 of this invention, respectively in combination with 0.17% of the known Compound (a) and 1% of the known Compound (s) were each prepared by using 1.5 parts of the compound of this invention, 0.17 part of

the known Compound (a), 1.0 part of the known Compound (s), 1 part of lauryl sulfate, 1 part of calcium lignin sulfonate, 30 parts of bentonite and 65.33 parts of white clay, mixing and formulating them in a manner similar to Example 11.

The compounds of the general formula (I) according to this invention have a herbicidal activity and safety superior to the similar herbicidal compounds known to date.

Namely, the compounds of this invention can act against a wide variety of weeds growing in the paddy fields of rice plant, for example, such weeds as barnyard grass (Echinochloa crus-galli), hardstem bulrush (Scirpus hotarui), narrowleaved waterplantain (Alisma canaliculatum), Monochoria (Monochoria vaginalis), false pimpernel (Lindernia procumbens), and toothcup (Rotala indica) and are able to kill completely these weeds at a rate of application of 50 g or less of the active ingredient per 10 ares. Furthermore, depending on the nature of the compounds of the present invention, some compounds can kill completely many sorts of the weeds growing in the paddy fields even when they are applied at a rate of application as low as 6.25 g or less per 10 ares. In addition, the compounds of this invention are able to kill completely a variety of weeds growing in the fields of upland crop plants, for example, such weeds as large crabgrass (Digitaria sanguinalis), green foxtail (Setaria viridus), common lambusquaters (Chenopodium album), livid amaranth (Amaranthus lividus) and smart weed (Polygonum blumei), when these weeds are treated with the compound of this invention at a rate of application of 100 g or less of the active ingredient per 10 ares. Besides, depending on the nature of the compounds of this invention as employed, some compounds can kill completely many sort of the weeds growing in the fields of upland crops even when they are applied at a rate of application as low as 12.5 g or less per 10 ares. Moreover, the compounds of this invention do not give phytotoxical damage to economical crops such as paddy rice plant, soybean, wheat, corn, beet, rapeseed plant and the like. The compounds of this invention have low toxicity to human and stock animals, as well as low toxicity to fishes and hence can be applied with safety.

To demonstrate the herbicidal activity of the compounds of the general formula (I) according to this invention, Test Examples 1-4 will hereinafter be described.

Test Example 1

Tests for estimation of herbicidal effects of the compound of this invention against weeds growing in paddy field of rice, and tests for estimation of phytotoxicity of the compound of this invention to transplanted paddy rice plant

Paddy field soil (alluvial soil) was placed in Wagner pots having a size of 1/5,000 ares. In the surface layers of the soil in these pots, there were evenly sowed at a population of 50 seeds per pot seeds of barnyard grass, hardstem bulrush, narrowleaved waterplantain, Monochoria, false pimpernel and toothcup, respectively, which are weeds ordinarily growing in the paddy field of rice plant. One day after the sowing of seeds, each pot was irrigated with water with the water depth being kept at 2 cm. Three days after the sowing of the weed seeds, paddy rice seedlings (at 2.5 leaf stage) were transplanted into each pot at a rate of 3 hills per pot with two rice seedlings being assembled in each hill. One day after the transplantation of the rice seedlings, a spraying test emulsion containing the compound of this invention to be tested was dropwise added into the irrigating water in each pot at a rate of 10 ml of said emulsion per pot, which was equivalent to a rate of application of 50 g of the active ingredient per 10 ares. Said spraying test emulsion had been prepared by diluting with water such an emulsifiable concentrate of the compound of this invention which was formulated similarly to the procedure of Example 19.

The above tests were repeated using each and all of Compound No. 1 to Compound No. 410 of this invention indicated in Table 1. These tests were repeated twice for each and every test compound in the same way and thus were conducted in two replicates for each test plot. 30 Days after the herbicidal treatment as made by the application of the test compound, the herbicidal effects of the test compounds and the degree of the phytotoxicity of the test compound to the paddy rice were estimated according to the following indexes of estimation:

| Index for estimation of herbicidal effects | Percentages (%) of kill of weed |
|---|---|
| 5 | 100% |
| 4 | 80% to less than 100% |
| 3 | 60% to less than 80% |
| 2 | 40% to less than 60% |
| 1 | 20% to less than 40% |
| 0 | Less than 20% |

| Index for phototoxicity | Degree of damage |
|---|---|
| 5 | Kill |
| 4 | Severe damage |
| 3 | Moderate damage |
| 2 | Slight damage |
| 1 | Minor damage |
| 0 | No damage |

The test results obtained are summarized in Table 3 below. All and each of Compound No. 1 to Compound No. 410 as tested afforded equally an estimated index of 5 for their herbicidal effects and also equally gave an estimated index of zero for their phytotoxicity to rice. For the sake of abbreviation and simplification, therefore, Table 3 only represents the test results which were obtained with some members chosen from amongst the whole test compounds according to this invention.

Table 3

| Test compound No. | Herbicidal effect | | | | | | Phytotoxicity to paddy rice plant |
|---|---|---|---|---|---|---|---|
| | Barnyard grass | Hardstem bulrush | Narrowleaved water plantain | Monochoria | False pimpernel | Toothcup | |
| 1 | 5 | 5 | 5 | 5 | 5 | 5 | 0 |
| ~ | ~ | ~ | ~ | ~ | ~ | ~ | ~ |
| 5 | 5 | 5 | 5 | 5 | 5 | 5 | 0 |
| ~ | ~ | ~ | ~ | ~ | ~ | ~ | ~ |
| 7 | 5 | 5 | 5 | 5 | 5 | 5 | 0 |
| ~ | ~ | ~ | ~ | ~ | ~ | ~ | ~ |
| 53 | 5 | 5 | 5 | 5 | 5 | 5 | 0 |
| ~ | ~ | ~ | ~ | ~ | ~ | ~ | ~ |
| 65 | 5 | 5 | 5 | 5 | 5 | 5 | 0 |
| 66 | 5 | 5 | 5 | 5 | 5 | 5 | 0 |
| ~ | ~ | ~ | ~ | ~ | ~ | ~ | ~ |
| 202 | 5 | 5 | 5 | 5 | 5 | 5 | 0 |
| ~ | ~ | ~ | ~ | ~ | ~ | ~ | ~ |
| 227 | 5 | 5 | 5 | 5 | 5 | 5 | 0 |
| ~ | ~ | ~ | ~ | ~ | ~ | ~ | ~ |
| 300 | 5 | 5 | 5 | 5 | 5 | 5 | 0 |
| ~ | ~ | ~ | ~ | ~ | ~ | ~ | ~ |
| ~ | ~ | ~ | ~ | ~ | ~ | ~ | ~ |
| 410 | 5 | 5 | 5 | 5 | 5 | 5 | 0 |

Table 3 (Continued)

| Test compound No. | Herbicidal effect | | | | | | Phytotoxicity to paddy rice plant |
|---|---|---|---|---|---|---|---|
| | Barnyard grass | Hardstem bulrush | Narrowleaved water plantain | Monochoria | False pimpernel | Toothcup | |
| Comparative agent A | 2 | 2 | 2 | 1 | 1 | 2 | 1 |
| Comparative agent B | 2 | 2 | 2 | 1 | 1 | 1 | 1 |
| Comparative agent C | 1 | 2 | 1 | 1 | 1 | 2 | 1 |
| Comparative agent D | 1 | 1 | 2 | 1 | 1 | 1 | 1 |
| Comparative agent E | 4 | 4 | 4 | 3 | 3 | 3 | 3 |

Comparative agent A:

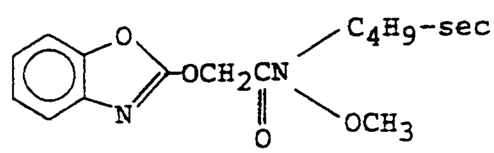

(Compound described in Japanese patent application first publication "Kokai" No. Sho-56-86176 specification and Japanese patent application first publication "Kokai" No. Sho-56-4903 specification)

Comparative agent B:

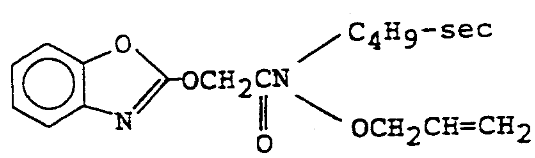

(Ditto)

Comparative agent C:

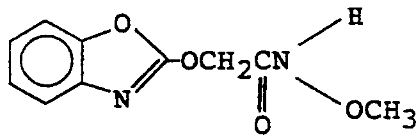

(Compound described in Japanese patent application first publication "Kokai" No. Sho-56-86176 specification)

Comparative agent D:

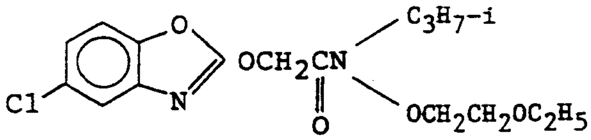

(Compound described in Japanese patent application first publication "Kokai" No. Sho-56-86176 specification)

Comparative agent E:

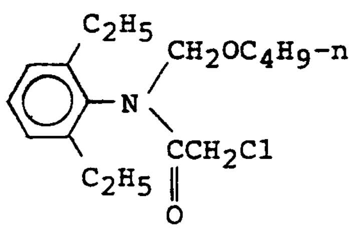

(Common name: butachlor)

Test Example 2

Test for estimation of herbicidal effects of the compound of this invention against weeds growing in paddy field of rice, and tests for estimation of phytotoxicity of the compound to transplanted paddy rice plant, upon applications of the compound at low rates

The procedure of Test Example 1 was followed, except that the amounts of the compounds of this invention applied were reduced to such lower rates of application of the active ingredient as indicated in Table 4 hereinafter. The herbicidal effects of the compounds of this invention against the weeds in the paddy field, as well as the degree of phytotoxicity of said compounds to transplanted paddy rice plants upon use of the compounds at their lower application rates were assessed in terms of the same indexes of estimation as defined in Test Example 1. Then, these tests were repeated twice in the same way for each test compound at one and same rate of application of the test compound. Thus, these tests were conducted in two replicates for each test plot. The test results obtained are shown in the following Table 4.

Table 4

| Test compound No. | Rate of application of compound (g/10 ares) | Herbicidal effect | | | | | | Phytotoxicity to paddy rice plant |
|---|---|---|---|---|---|---|---|---|
| | | Barnyard grass | Hardstem bulrush | Narrowleaved water plantain | Monochoria | False pimpernel | Toothcup | |
| 5 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 8 | 25 | 5 | 5 | 5 | 5 | 5 | 5 | 0 |
| | 12.5 | 5 | 5 | 5 | 5 | 5 | 5 | 0 |
| | 6.25 | 4 | 4 | 5 | 4 | 5 | 5 | 0 |
| 10 | 25 | 5 | 5 | 5 | 5 | 5 | 5 | 0 |
| | 12.5 | 5 | 5 | 5 | 5 | 5 | 5 | 0 |
| | 6.25 | 5 | 5 | 4 | 4 | 5 | 5 | 0 |
| 11 | 25 | 5 | 5 | 5 | 5 | 5 | 5 | 0 |
| | 12.5 | 5 | 5 | 5 | 5 | 5 | 5 | 0 |
| | 6.25 | 5 | 4 | 4 | 5 | 5 | 5 | 0 |
| 13 | 25 | 5 | 5 | 5 | 5 | 5 | 5 | 0 |
| | 12.5 | 5 | 4 | 5 | 4 | 5 | 5 | 0 |
| | 6.25 | 4 | 4 | 4 | 4 | 5 | 5 | 0 |
| 15 | 25 | 5 | 5 | 5 | 5 | 5 | 5 | 0 |
| | 12.5 | 5 | 5 | 5 | 5 | 5 | 5 | 0 |
| | 6.25 | 4 | 4 | 4 | 4 | 5 | 5 | 0 |
| 16 | 25 | 5 | 5 | 5 | 5 | 5 | 5 | 0 |
| | 12.5 | 5 | 5 | 5 | 4 | 5 | 5 | 0 |
| | 6.25 | 4 | 4 | 4 | 3 | 4 | 5 | 0 |
| 21 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 4 | 5 | 5 | 0 |
| 23 | 25 | 5 | 5 | 5 | 5 | 5 | 5 | 0 |
| | 12.5 | 5 | 5 | 5 | 5 | 5 | 5 | 0 |
| | 6.25 | 5 | 4 | 5 | 4 | 5 | 5 | 0 |
| 28 | 25 | 5 | 5 | 5 | 5 | 5 | 5 | 0 |
| | 12.5 | 5 | 5 | 5 | 5 | 5 | 5 | 0 |
| | 6.25 | 4 | 5 | 5 | 5 | 5 | 4 | 0 |
| 41 | 25 | 5 | 5 | 5 | 5 | 5 | 5 | 0 |
| | 12.5 | 5 | 5 | 5 | 5 | 5 | 5 | 0 |
| | 6.25 | 5 | 5 | 4 | 5 | 5 | 5 | 0 |
| 42 | 25 | 5 | 5 | 5 | 5 | 5 | 5 | 0 |
| | 12.5 | 5 | 5 | 5 | 5 | 5 | 5 | 0 |
| | 6.25 | 5 | 4 | 5 | 5 | 5 | 5 | 0 |

Table 4
(Continued)

| Test compound No. | Rate of application of compound (g/10 ares) | Herbicidal effect | | | | | | Phytotoxicity to paddy rice plant |
|---|---|---|---|---|---|---|---|---|
| | | Barnyard grass | Hardstem bulrush | Narrowleaved water plantain | Monochoria | False pimpernel | Toothcup | |
| 45 | 25 | 5 | 5 | 5 | 5 | 5 | 5 | 0 |
| | 12.5 | 5 | 5 | 5 | 5 | 5 | 5 | 0 |
| | 6.25 | 4 | 4 | 5 | 4 | 5 | 4 | 0 |
| 47 | 25 | 5 | 5 | 5 | 5 | 5 | 5 | 0 |
| | 12.5 | 5 | 5 | 4 | 4 | 5 | 5 | 0 |
| | 6.25 | 5 | 4 | 4 | 4 | 5 | 5 | 0 |
| 53 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 56 | 25 | 5 | 5 | 5 | 5 | 5 | 5 | 0 |
| | 12.5 | 5 | 5 | 5 | 5 | 5 | 5 | 0 |
| | 6.25 | 4 | 5 | 4 | 4 | 5 | 5 | 0 |
| 65 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 67 | 25 | 5 | 5 | 5 | 5 | 5 | 5 | 0 |
| | 12.5 | 5 | 5 | 5 | 5 | 5 | 5 | 0 |
| | 6.25 | 5 | 5 | 4 | 4 | 5 | 5 | 0 |
| 79 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 4 | 5 | 5 | 0 |
| 84 | 25 | 5 | 5 | 5 | 5 | 5 | 5 | 0 |
| | 12.5 | 5 | 5 | 5 | 5 | 5 | 5 | 0 |
| | 6.25 | 5 | 4 | 5 | 4 | 5 | 5 | 0 |
| 89 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 90 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 91 | 25 | 5 | 5 | 5 | 5 | 5 | 5 | 0 |
| | 12.5 | 5 | 5 | 4 | 5 | 5 | 5 | 0 |
| | 6.25 | 4 | 4 | 4 | 4 | 5 | 5 | 0 |
| 94 | 25 | 5 | 5 | 5 | 5 | 5 | 5 | 0 |
| | 12.5 | 5 | 4 | 4 | 5 | 5 | 5 | 0 |
| | 6.25 | 4 | 4 | 4 | 4 | 4 | 5 | 0 |

Table 4
(Continued)

| Test compound No. | Rate of application of compound (g/10 ares) | Herbicidal effect | | | | | | Phytotoxicity to paddy rice plant |
|---|---|---|---|---|---|---|---|---|
| | | Barnyard grass | Hardstem bulrush | Narrowleaved water plantain | Monochoria | False pimpernel | Toothcup | |
| 95 | 25 | 5 | 5 | 5 | 5 | 5 | 5 | 0 |
| | 12.5 | 5 | 5 | 5 | 5 | 5 | 5 | 0 |
| | 6.25 | 5 | 5 | 4 | 4 | 4 | 4 | 0 |
| 98 | 25 | 5 | 5 | 5 | 5 | 5 | 5 | 0 |
| | 12.5 | 5 | 5 | 5 | 5 | 5 | 5 | 0 |
| | 6.25 | 5 | 4 | 4 | 5 | 4 | 5 | 0 |
| 105 | 25 | 5 | 5 | 5 | 5 | 5 | 5 | 0 |
| | 12.5 | 5 | 5 | 5 | 5 | 5 | 5 | 0 |
| | 6.25 | 5 | 5 | 4 | 4 | 5 | 5 | 0 |
| 113 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 4 | 5 | 5 | 0 |
| 118 | 25 | 5 | 5 | 5 | 5 | 5 | 5 | 0 |
| | 12.5 | 5 | 5 | 5 | 5 | 5 | 5 | 0 |
| | 6.25 | 4 | 5 | 4 | 4 | 5 | 5 | 0 |
| 122 | 25 | 5 | 5 | 5 | 5 | 5 | 5 | 0 |
| | 12.5 | 5 | 5 | 5 | 5 | 5 | 5 | 0 |
| | 6.25 | 5 | 5 | 4 | 5 | 5 | 5 | 0 |
| 126 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 129 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 130 | 25 | 5 | 5 | 5 | 5 | 5 | 5 | 0 |
| | 12.5 | 4 | 4 | 4 | 4 | 5 | 4 | 0 |
| | 6.25 | 4 | 4 | 4 | 4 | 5 | 4 | 0 |
| 133 | 25 | 5 | 5 | 5 | 5 | 5 | 5 | 0 |
| | 12.5 | 5 | 5 | 5 | 5 | 5 | 5 | 0 |
| | 6.25 | 5 | 4 | 5 | 5 | 5 | 5 | 0 |
| 135 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 4 | 5 | 5 | 0 |
| 138 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 4 | 5 | 5 | 0 |

Table 4
(Continued)

| Test compound No. | Rate of application of compound (g/10 ares) | Herbicidal effect | | | | | | Phytotoxicity to paddy rice plant |
|---|---|---|---|---|---|---|---|---|
| | | Barnyard grass | Hardstem bulrush | Narrowleaved water plantain | Monochoria | False pimpernel | Toothcup | |
| 144 | 25 | 5 | 5 | 5 | 5 | 5 | 5 | 0 |
| | 12.5 | 5 | 5 | 5 | 5 | 5 | 5 | 0 |
| | 6.25 | 5 | 4 | 5 | 5 | 5 | 5 | 0 |
| 151 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 153 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 165 | 25 | 5 | 5 | 5 | 5 | 5 | 5 | 0 |
| | 12.5 | 5 | 5 | 5 | 5 | 5 | 5 | 0 |
| | 6.25 | 5 | 4 | 4 | 4 | 5 | 5 | 0 |
| 170 | 25 | 5 | 5 | 5 | 5 | 5 | 5 | 0 |
| | 12.5 | 5 | 5 | 5 | 5 | 5 | 5 | 0 |
| | 6.25 | 4 | 4 | 4 | 4 | 5 | 5 | 0 |
| 176 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 178 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 182 | 25 | 5 | 5 | 5 | 5 | 5 | 5 | 0 |
| | 12.5 | 5 | 5 | 5 | 5 | 5 | 5 | 0 |
| | 6.25 | 5 | 5 | 4 | 5 | 5 | 5 | 0 |
| 184 | 25 | 5 | 5 | 5 | 5 | 5 | 5 | 0 |
| | 12.5 | 5 | 5 | 5 | 5 | 5 | 5 | 0 |
| | 6.25 | 5 | 5 | 4 | 5 | 5 | 5 | 0 |
| 194 | 25 | 5 | 5 | 5 | 5 | 5 | 5 | 0 |
| | 12.5 | 5 | 5 | 5 | 5 | 5 | 5 | 0 |
| | 6.25 | 4 | 5 | 4 | 5 | 5 | 5 | 0 |
| 202 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 207 | 25 | 5 | 5 | 5 | 5 | 5 | 5 | 0 |
| | 12.5 | 5 | 5 | 5 | 5 | 5 | 5 | 0 |
| | 6.25 | 4 | 4 | 4 | 5 | 5 | 5 | 0 |

Table 4
(Continued)

| Test compound No. | Rate of application of compound (g/10 ares) | Herbicidal effect | | | | | | Phytotoxicity to paddy rice plant |
|---|---|---|---|---|---|---|---|---|
| | | Barnyard grass | Hardstem bulrush | Narrowleaved water plantain | Monochoria | False pimpernel | Toothcup | |
| 220 | 25 | 5 | 5 | 5 | 5 | 5 | 5 | 0 |
| | 12.5 | 5 | 5 | 5 | 5 | 5 | 5 | 0 |
| | 6.25 | 5 | 5 | 4 | 5 | 5 | 5 | 0 |
| 224 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 228 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 232 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 234 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 239 | 25 | 5 | 5 | 5 | 5 | 5 | 5 | 0 |
| | 12.5 | 5 | 5 | 5 | 5 | 5 | 5 | 0 |
| | 6.25 | 5 | 4 | 5 | 5 | 5 | 5 | 0 |
| 246 | 25 | 5 | 5 | 5 | 5 | 5 | 5 | 0 |
| | 12.5 | 5 | 5 | 5 | 5 | 5 | 5 | 0 |
| | 6.25 | 5 | 4 | 5 | 5 | 5 | 5 | 0 |
| 248 | 25 | 5 | 5 | 5 | 5 | 5 | 5 | 0 |
| | 12.5 | 5 | 5 | 5 | 5 | 5 | 5 | 0 |
| | 6.25 | 5 | 4 | 5 | 5 | 5 | 5 | 0 |
| 253 | 25 | 5 | 5 | 5 | 5 | 5 | 5 | 0 |
| | 12.5 | 5 | 5 | 5 | 5 | 5 | 5 | 0 |
| | 6.25 | 4 | 4 | 5 | 5 | 5 | 5 | 0 |
| 254 | 25 | 5 | 5 | 5 | 5 | 5 | 5 | 0 |
| | 12.5 | 5 | 5 | 5 | 5 | 5 | 5 | 0 |
| | 6.25 | 5 | 5 | 4 | 4 | 4 | 4 | 0 |
| 260 | 25 | 5 | 5 | 5 | 5 | 5 | 5 | 0 |
| | 12.5 | 5 | 5 | 4 | 5 | 5 | 5 | 0 |
| | 6.25 | 4 | 4 | 4 | 5 | 5 | 5 | 0 |
| 261 | 25 | 5 | 5 | 5 | 5 | 5 | 5 | 0 |
| | 12.5 | 5 | 5 | 5 | 5 | 5 | 5 | 0 |
| | 6.25 | 5 | 5 | 4 | 5 | 5 | 5 | 0 |

Table 4
(Continued)

| Test compound No. | Rate of application of compound (g/10 ares) | Herbicidal effect | | | | | | Phytotoxicity to paddy rice plant |
|---|---|---|---|---|---|---|---|---|
| | | Barnyard grass | Hardstem bulrush | Narrowleaved water plantain | Monochoria | False pimpernel | Toothcup | |
| 263 | 25 | 5 | 5 | 5 | 5 | 5 | 5 | 0 |
| | 12.5 | 4 | 4 | 5 | 4 | 5 | 5 | 0 |
| | 6.25 | 4 | 4 | 4 | 4 | 4 | 5 | 0 |
| 264 | 25 | 5 | 5 | 5 | 5 | 5 | 5 | 0 |
| | 12.5 | 5 | 5 | 5 | 5 | 5 | 5 | 0 |
| | 6.25 | 5 | 4 | 4 | 5 | 5 | 5 | 0 |
| 266 | 25 | 5 | 5 | 5 | 5 | 5 | 5 | 0 |
| | 12.5 | 5 | 5 | 5 | 5 | 5 | 5 | 0 |
| | 6.25 | 4 | 4 | 5 | 4 | 5 | 5 | 0 |
| 271 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 277 | 25 | 5 | 5 | 5 | 5 | 5 | 5 | 0 |
| | 12.5 | 5 | 5 | 5 | 5 | 5 | 5 | 0 |
| | 6.25 | 4 | 4 | 4 | 4 | 5 | 5 | 0 |
| 284 | 25 | 5 | 5 | 5 | 5 | 5 | 5 | 0 |
| | 12.5 | 5 | 5 | 5 | 5 | 5 | 5 | 0 |
| | 6.25 | 5 | 4 | 4 | 5 | 5 | 5 | 0 |
| 291 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 294 | 25 | 5 | 5 | 5 | 5 | 5 | 5 | 0 |
| | 12.5 | 5 | 5 | 5 | 5 | 5 | 5 | 0 |
| | 6.25 | 5 | 4 | 5 | 5 | 5 | 5 | 0 |
| 297 | 25 | 5 | 5 | 5 | 5 | 5 | 5 | 0 |
| | 12.5 | 5 | 5 | 5 | 5 | 5 | 5 | 0 |
| | 6.25 | 5 | 5 | 4 | 5 | 5 | 5 | 0 |
| 301 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 303 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 305 | 25 | 5 | 5 | 5 | 5 | 5 | 5 | 0 |
| | 12.5 | 5 | 5 | 5 | 5 | 5 | 5 | 0 |
| | 6.25 | 5 | 4 | 5 | 5 | 5 | 5 | 0 |

Table 4
(Continued)

| Test compound No. | Rate of application of compound (g/10 ares) | Herbicidal effect | | | | | | Phytotoxicity to paddy rice plant |
|---|---|---|---|---|---|---|---|---|
| | | Barnyard grass | Hardstem bulrush | Narrowleaved water plantain | Monochoria | False pimpernel | Toothcup | |
| 309 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 312 | 25 | 5 | 5 | 5 | 5 | 5 | 5 | 0 |
| | 12.5 | 5 | 5 | 5 | 5 | 5 | 5 | 0 |
| | 6.25 | 4 | 4 | 5 | 5 | 5 | 5 | 0 |
| 315 | 25 | 5 | 5 | 5 | 5 | 5 | 5 | 0 |
| | 12.5 | 5 | 4 | 5 | 5 | 5 | 5 | 0 |
| | 6.25 | 4 | 4 | 5 | 5 | 5 | 5 | 0 |
| 324 | 25 | 5 | 5 | 5 | 5 | 5 | 5 | 0 |
| | 12.5 | 4 | 5 | 4 | 5 | 4 | 5 | 0 |
| | 6.25 | 4 | 4 | 4 | 4 | 3 | 4 | 0 |
| 329 | 25 | 5 | 5 | 5 | 5 | 5 | 5 | 0 |
| | 12.5 | 4 | 4 | 4 | 4 | 4 | 4 | 0 |
| | 6.25 | 4 | 3 | 4 | 4 | 3 | 4 | 0 |
| 332 | 25 | 5 | 5 | 5 | 5 | 5 | 5 | 0 |
| | 12.5 | 4 | 5 | 4 | 4 | 5 | 5 | 0 |
| | 6.25 | 4 | 4 | 3 | 3 | 4 | 4 | 0 |
| 335 | 25 | 5 | 5 | 5 | 5 | 5 | 5 | 0 |
| | 12.5 | 5 | 4 | 5 | 4 | 5 | 5 | 0 |
| | 6.25 | 4 | 3 | 4 | 3 | 4 | 5 | 0 |
| 340 | 25 | 5 | 5 | 5 | 5 | 5 | 5 | 0 |
| | 12.5 | 5 | 5 | 5 | 5 | 5 | 5 | 0 |
| | 6.25 | 4 | 4 | 5 | 5 | 5 | 5 | 0 |
| 344 | 25 | 5 | 5 | 5 | 5 | 5 | 5 | 0 |
| | 12.5 | 5 | 5 | 5 | 5 | 5 | 5 | 0 |
| | 6.25 | 4 | 4 | 4 | 5 | 4 | 5 | 0 |
| 353 | 25 | 5 | 5 | 5 | 5 | 5 | 5 | 0 |
| | 12.5 | 5 | 4 | 5 | 5 | 5 | 5 | 0 |
| | 6.25 | 4 | 3 | 4 | 4 | 4 | 4 | 0 |
| 366 | 25 | 5 | 5 | 5 | 5 | 5 | 5 | 0 |
| | 12.5 | 5 | 5 | 5 | 5 | 5 | 5 | 0 |
| | 6.25 | 5 | 5 | 4 | 4 | 5 | 5 | 0 |
| 367 | 25 | 5 | 5 | 5 | 5 | 5 | 5 | 0 |
| | 12.5 | 5 | 5 | 5 | 5 | 5 | 5 | 0 |
| | 6.25 | 5 | 5 | 4 | 5 | 5 | 5 | 0 |

Table 4
(Continued)

| Test compound No. | Rate of application of compound (g/10 ares) | Herbicidal effect | | | | | | Phytotoxicity to paddy rice plant |
|---|---|---|---|---|---|---|---|---|
| | | Barnyard grass | Hardstem bulrush | Narrowleaved water plantain | Monochoria | False pimpernel | Toothcup | |
| 370 | 25 | 5 | 5 | 5 | 5 | 5 | 5 | 0 |
| | 12.5 | 5 | 5 | 5 | 5 | 5 | 5 | 0 |
| | 6.25 | 5 | 4 | 5 | 5 | 5 | 5 | 0 |
| 373 | 25 | 5 | 5 | 5 | 5 | 5 | 5 | 0 |
| | 12.5 | 5 | 5 | 5 | 5 | 4 | 5 | 0 |
| | 6.25 | 4 | 4 | 4 | 5 | 4 | 4 | 0 |
| 381 | 25 | 5 | 5 | 5 | 5 | 5 | 5 | 0 |
| | 12.5 | 5 | 5 | 5 | 5 | 5 | 5 | 0 |
| | 6.25 | 5 | 4 | 5 | 5 | 5 | 5 | 0 |
| 383 | 25 | 5 | 5 | 5 | 5 | 5 | 5 | 0 |
| | 12.5 | 5 | 5 | 5 | 5 | 5 | 5 | 0 |
| | 6.25 | 5 | 4 | 5 | 5 | 5 | 5 | 0 |
| 390 | 25 | 5 | 5 | 5 | 5 | 5 | 5 | 0 |
| | 12.5 | 4 | 5 | 5 | 4 | 5 | 5 | 0 |
| | 6.25 | 3 | 4 | 4 | 3 | 5 | 4 | 0 |
| 393 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 394 | 25 | 5 | 5 | 5 | 5 | 5 | 5 | 0 |
| | 12.5 | 5 | 5 | 5 | 5 | 5 | 5 | 0 |
| | 6.25 | 5 | 5 | 5 | 5 | 5 | 5 | 0 |
| 402 | 25 | 5 | 5 | 5 | 5 | 5 | 5 | 0 |
| | 12.5 | 5 | 5 | 5 | 5 | 5 | 5 | 0 |
| | 6.25 | 4 | 4 | 5 | 5 | 5 | 5 | 0 |
| 405 | 25 | 5 | 5 | 5 | 5 | 5 | 5 | 0 |
| | 12.5 | 5 | 5 | 5 | 5 | 5 | 5 | 0 |
| | 6.25 | 4 | 5 | 4 | 5 | 5 | 5 | 0 |
| 409 | 25 | 5 | 5 | 5 | 5 | 5 | 5 | 0 |
| | 12.5 | 4 | 5 | 5 | 5 | 5 | 5 | 0 |
| | 6.25 | 3 | 4 | 4 | 5 | 4 | 5 | 0 |
| Comparative agent A | 25 | 1 | 1 | 1 | 0 | 0 | 1 | 1 |
| | 12.5 | 1 | 0 | 1 | 0 | 0 | 0 | 0 |
| | 6.25 | 0 | 0 | 0 | 0 | 0 | 0 | 0 |
| Comparative agent B | 25 | 1 | 1 | 1 | 0 | 0 | 0 | 0 |
| | 12.5 | 0 | 1 | 0 | 0 | 0 | 0 | 0 |
| | 6.25 | 0 | 0 | 0 | 0 | 0 | 0 | 0 |

Table 4
(Continued)

| Test compound No. | Rate of application of compound (g/10 ares) | Herbicidal effect | | | | | | Phytotoxicity to paddy rice plant |
|---|---|---|---|---|---|---|---|---|
| | | Barnyard grass | Hardstem bulrush | Narrowleaved water plantain | Monochoria | False pimpernel | Toothcup | |
| Comparative agent C | 25 | 0 | 0 | 1 | 0 | 0 | 1 | 0 |
| | 12.5 | 0 | 0 | 0 | 0 | 0 | 0 | 0 |
| | 6.25 | 0 | 0 | 0 | 0 | 0 | 0 | 0 |
| Comparative agent D | 25 | 0 | 0 | 1 | 0 | 0 | 0 | 0 |
| | 12.5 | 0 | 0 | 0 | 0 | 0 | 0 | 0 |
| | 6.25 | 0 | 0 | 0 | 0 | 0 | 0 | 0 |
| Comparative agent E | 25 | 3 | 3 | 4 | 3 | 3 | 3 | 3 |
| | 12.5 | 2 | 2 | 3 | 3 | 3 | 1 | 3 |
| | 6.25 | 2 | 1 | 1 | 1 | 0 | 1 | 1 |

Note: Comparative agents A to E are the same as those shown in Table 3.

Test Example 3

Tests for estimation of herbicidal effects of the compound of this invention against weeds growing in fields of upland crops, and tests for estimation of phytotoxicity of the compound to various sorts of upland crop plants

(1) Tests for estimation of herbicidal effects

An amount of a soil of the upland field (alluvial soil) was placed in Wagner pots having a size of 1/5,000 ares. Seeds of a weed were sown at a population of 50 seeds per pot into the soil by mixing evenly the seeds with the soil of the surface layer of the soil present to a depth of 1 cm from the top of the soil in each pot, followed by gently pressing on the surface layer of the soil in the pots. The seeds so sown in the pots were respectively those of various sorts of weeds which ordinarily grow in the fields of upland crops and which were seeds of large crabgrass, green foxtail, common lambusquaters, livid amaranth and smart weed. Two days after the sowing of seeds, a spraying test emulsion containing the compound of this invention was sprayed over the surface of the soil in the pots at a rate of application of 100 litres of said test emulsion per 10 ares, which was corresponding to a rate of application of 100 g of the active ingredient per 10 ares. The test emulsion so sprayed had been prepared by diluting with water such an emulsifiable concentrate of the compound of this invention which had been formulated in the same manner as in Example 19 hereinbefore. The above tests were repeated using each and all of Compound No. 1 to Compound No. 410 of this invention indicated in Table 1 hereinbefore. These tests were repeated twice in the same way for each test compound. Namely, these tests were conducted in two replicates for each test plot. 30 Days after the herbicidal treatment as made by application of the test compound, the herbicidal effects of the test compound applied were assessed in terms of the indexes of estimation as shown in Test Example 1.

(2) Test for phytotoxicity to crop plants

An amount of a soil of the upland field (alluvial soil) was placed in Wagner pots having a size of 1/10,000 ares. Into the surface layer of the soil in these pots, there were sown seeds of each of various

sorts of crop plants (at populations of 5 seeds for soybean, 5 seeds for corn, 10 seeds for beet, 10 seeds for rapeseed grass and 10 seeds for wheat, respectively in the separate pots), followed by gently pressing on the surface layer of the soil in the pots.

One day after the sowing of seeds, a spraying test emulsion containing the compound of this invention was sprayed over the surface of the soil in the pots at a rate of application of 100 litres of said test emulsion per 10 ares, which was corresponding to a rate of application of 100 g of the active ingredient per 10 ares. The test emulsion so sprayed had been prepared by diluting with water such an emulsifiable concentrate of the compound of this invention which had been formulated in the same manner as in Example 19 hereinbefore.

The above tests were repeated twice in the same way for each test compound and thus these tests were conducted in two replicates for each test plot. 30 Days after the application of the test compound, the degree of phytotoxicity of the test compound to the various crop plants were assessed in terms of the indexes of estimation as shown in Test Example 1.

The test results obtained are summarized in Table 5 below.

All and each of Compound No. 1 to Compound No. 410 as tested afforded equally an estimated index of 5 for their herbicidal effects and also equally gave an estimated index of zero for their phytotoxicity to crops. For the sake of abbreviation and simplification, therefore, Table 5 only represents the test results which were obtained with some members chosen from amongst the whole test compounds according to this invention.

Table 5

| Test compound No. | Herbicidal effect against upland weeds | | | | | Phytotoxicity to crop plants | | | | |
|---|---|---|---|---|---|---|---|---|---|---|
| | Large crabgrass | Green foxtail | Common lambusquaters | Livid amaranth | Smartweed | Soybean | Corn | Beet | Rapeseed | Wheat |
| 1 | 5 | 5 | 5 | 5 | 5 | 0 | 0 | 0 | 0 | 0 |
| ⌇ | ⌇ | ⌇ | ⌇ | ⌇ | ⌇ | ⌇ | ⌇ | ⌇ | ⌇ | ⌇ |
| 5 | 5 | 5 | 5 | 5 | 5 | 0 | 0 | 0 | 0 | 0 |
| ⌇ | ⌇ | ⌇ | ⌇ | ⌇ | ⌇ | ⌇ | ⌇ | ⌇ | ⌇ | ⌇ |
| 7 | 5 | 5 | 5 | 5 | 5 | 0 | 0 | 0 | 0 | 0 |
| ⌇ | ⌇ | ⌇ | ⌇ | ⌇ | ⌇ | ⌇ | ⌇ | ⌇ | ⌇ | ⌇ |
| 53 | 5 | 5 | 5 | 5 | 5 | 0 | 0 | 0 | 0 | 0 |
| ⌇ | ⌇ | ⌇ | ⌇ | ⌇ | ⌇ | ⌇ | ⌇ | ⌇ | ⌇ | ⌇ |
| 65 | 5 | 5 | 5 | 5 | 5 | 0 | 0 | 0 | 0 | 0 |
| 66 | 5 | 5 | 5 | 5 | 5 | 0 | 0 | 0 | 0 | 0 |
| ⌇ | ⌇ | ⌇ | ⌇ | ⌇ | ⌇ | ⌇ | ⌇ | ⌇ | ⌇ | ⌇ |
| 202 | 5 | 5 | 5 | 5 | 5 | 0 | 0 | 0 | 0 | 0 |
| ⌇ | ⌇ | ⌇ | ⌇ | ⌇ | ⌇ | ⌇ | ⌇ | ⌇ | ⌇ | ⌇ |
| 227 | 5 | 5 | 5 | 5 | 5 | 0 | 0 | 0 | 0 | 0 |
| ⌇ | ⌇ | ⌇ | ⌇ | ⌇ | ⌇ | ⌇ | ⌇ | ⌇ | ⌇ | ⌇ |
| 300 | 5 | 5 | 5 | 5 | 5 | 0 | 0 | 0 | 0 | 0 |
| ⌇ | ⌇ | ⌇ | ⌇ | ⌇ | ⌇ | ⌇ | ⌇ | ⌇ | ⌇ | ⌇ |
| 410 | 5 | 5 | 5 | 5 | 5 | 0 | 0 | 0 | 0 | 0 |
| Comparative agent A | 2 | 2 | 1 | 3 | 1 | 3 | 2 | 1 | 3 | 2 |
| Comparative agent B | 2 | 2 | 1 | 2 | 1 | 2 | 2 | 1 | 3 | 2 |
| Comparative agent C | 2 | 1 | 1 | 2 | 1 | 1 | 1 | 0 | 2 | 1 |
| Comparative agent D | 1 | 1 | 1 | 2 | 1 | 1 | 1 | 0 | 2 | 0 |
| Comparative agent F | 4 | 3 | 4 | 4 | 4 | 2 | 0 | 1 | 3 | 1 |

Comparative agent F:

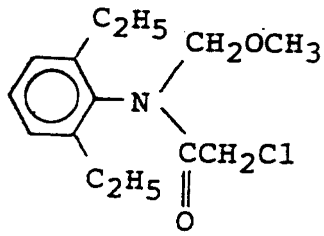

(Common name: alachlor)

Note): Comparative agent A to Comparative agent D are the same as those shown for Table 3.

Test Example 4

Test for estimation of herbicidal effects of the compound of this invention against weeds growing in fields of upland crops at low rates of application of the compound, and tests for estimation of phototoxicity of the compound to upland crop plants upon application of the compound at low rates

The procedure of Test Example 3 was followed, except that the amounts of the compounds of this invention applied were reduced to such lower rates of application of the active ingredient than in Test Example 3, such that the test compounds were applied at the rates indicated in Table 6 hereinafter.

The herbicidal effects of the compounds of this invention against the weeds growing the fields of upland crops, as well as the degree of phytotoxicity of the compounds to various upland crop plants upon application of the compounds at their low rates were assessed in terms of the same indexes of estimation as defined in Test Example 1. In this Test Example, the tests were again conducted in two replicates for each test plot.

The test results obtained are shown in the following Table 6.

Table 6

| Test compound No. | Rate of application of compound (g/10 ares) | Herbicidal effect against upland weeds | | | | | Phytotoxicity to crop plants | | | | |
|---|---|---|---|---|---|---|---|---|---|---|---|
| | | Large crabgrass | Green foxtail | Common lambusquaters | Livid amaranth | Smartweed | Soybean | Corn | Beet | Rapeseed | Wheat |
| 5 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 8 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 4 | 5 | 4 | 0 | 0 | 0 | 0 | 0 |
| 10 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 11 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 4 | 5 | 4 | 5 | 5 | 0 | 0 | 0 | 0 | 0 |
| 13 | 50 | 5 | 5 | 5 | 5 | 5 | 0 | 0 | 0 | 0 | 0 |
| | 25 | 5 | 4 | 4 | 5 | 4 | 0 | 0 | 0 | 0 | 0 |
| | 12.5 | 4 | 3 | 3 | 5 | 4 | 0 | 0 | 0 | 0 | 0 |
| 15 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 4 | 5 | 4 | 5 | 4 | 0 | 0 | 0 | 0 | 0 |
| 16 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 5 | 5 | 4 | 0 | 0 | 0 | 0 | 0 |
| 21 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 23 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 4 | 5 | 4 | 0 | 0 | 0 | 0 | 0 |

Table 6 (Continued)

| Test compound No. | Rate of application of compound (g/10 ares) | Herbicidal effect against upland weeds | | | | | Phytotoxicity to crop plants | | | | |
|---|---|---|---|---|---|---|---|---|---|---|---|
| | | Large crabgrass | Green foxtail | Common lambusquaters | Livid amaranth | Smartweed | Soybean | Corn | Beet | Rapeseed | Wheat |
| 28 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 41 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 4 | 5 | 5 | 0 | 0 | 0 | 0 | 0 |
| 42 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 4 | 5 | 4 | 0 | 0 | 0 | 0 | 0 |
| 45 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 4 | 5 | 4 | 0 | 0 | 0 | 0 | 0 |
| 47 | 50 | 5 | 5 | 5 | 5 | 5 | 0 | 0 | 0 | 0 | 0 |
| | 25 | 5 | 5 | 4 | 5 | 5 | 0 | 0 | 0 | 0 | 0 |
| | 12.5 | 4 | 4 | 4 | 4 | 4 | 0 | 0 | 0 | 0 | 0 |
| 53 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 56 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 5 | 5 | 5 | 0 | 0 | 0 | 0 | 0 |
| 65 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 67 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 4 | 5 | 5 | 0 | 0 | 0 | 0 | 0 |

Table 6 (Continued)

| Test compound No. | Rate of application of compound (g/10 ares) | Herbicidal effect against upland weeds | | | | | Phytotoxicity to crop plants | | | | |
|---|---|---|---|---|---|---|---|---|---|---|---|
| | | Large crabgrass | Green foxtail | Common lambusquaters | Livid amaranth | Smartweed | Soybean | Corn | Beet | Rapeseed | Wheat |
| 79 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 4 | 5 | 5 | 0 | 0 | 0 | 0 | 0 |
| 84 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 89 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 90 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 4 | 4 | 5 | 5 | 4 | 0 | 0 | 0 | 0 | 0 |
| 91 | 50 | 5 | 5 | 5 | 5 | 5 | 0 | 0 | 0 | 0 | 0 |
| | 25 | 4 | 4 | 5 | 5 | 4 | 0 | 0 | 0 | 0 | 0 |
| | 12.5 | 3 | 4 | 4 | 5 | 4 | 0 | 0 | 0 | 0 | 0 |
| 94 | 50 | 5 | 5 | 5 | 5 | 5 | 0 | 0 | 0 | 0 | 0 |
| | 25 | 5 | 4 | 5 | 4 | 4 | 0 | 0 | 0 | 0 | 0 |
| | 12.5 | 4 | 3 | 4 | 3 | 3 | 0 | 0 | 0 | 0 | 0 |
| 95 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 4 | 5 | 4 | 0 | 0 | 0 | 0 | 0 |
| 98 | 50 | 5 | 5 | 5 | 5 | 5 | 0 | 0 | 0 | 0 | 0 |
| | 25 | 4 | 4 | 5 | 5 | 4 | 0 | 0 | 0 | 0 | 0 |
| | 12.5 | 3 | 3 | 4 | 4 | 3 | 0 | 0 | 0 | 0 | 0 |
| 105 | 50 | 5 | 5 | 5 | 5 | 5 | 0 | 0 | 0 | 0 | 0 |
| | 25 | 5 | 5 | 4 | 5 | 4 | 0 | 0 | 0 | 0 | 0 |
| | 12.5 | 4 | 5 | 4 | 4 | 3 | 0 | 0 | 0 | 0 | 0 |

Table 6 (Continued)

| Test compound No. | Rate of application of compound (g/10 ares) | Herbicidal effect against upland weeds | | | | | Phytotoxicity to crop plants | | | | |
|---|---|---|---|---|---|---|---|---|---|---|---|
| | | Large crabgrass | Green foxtail | Common lambsquaters | Livid amaranth | Smartweed | Soybean | Corn | Beet | Rapeseed | Wheat |
| 113 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 4 | 5 | 0 | 0 | 0 | 0 | 0 |
| 118 | 50 | 5 | 5 | 5 | 5 | 5 | 0 | 0 | 0 | 0 | 0 |
| | 25 | 5 | 5 | 5 | 5 | 4 | 0 | 0 | 0 | 0 | 0 |
| | 12.5 | 4 | 4 | 4 | 4 | 4 | 0 | 0 | 0 | 0 | 0 |
| 122 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 126 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 129 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 130 | 50 | 5 | 5 | 5 | 5 | 5 | 0 | 0 | 0 | 0 | 0 |
| | 25 | 5 | 4 | 5 | 5 | 4 | 0 | 0 | 0 | 0 | 0 |
| | 12.5 | 4 | 3 | 4 | 4 | 3 | 0 | 0 | 0 | 0 | 0 |
| 133 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 4 | 5 | 4 | 0 | 0 | 0 | 0 | 0 |
| 135 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 4 | 5 | 4 | 0 | 0 | 0 | 0 | 0 |
| 138 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 4 | 5 | 4 | 0 | 0 | 0 | 0 | 0 |

Table 6 (Continued)

| Test compound No. | Rate of application of compound (g/10 ares) | Herbicidal effect against upland weeds | | | | | Phytotoxicity to crop plants | | | | |
|---|---|---|---|---|---|---|---|---|---|---|---|
| | | Large crabgrass | Green foxtail | Common lambusquaters | Livid amaranth | Smartweed | Soybean | Corn | Beet | Rapeseed | Wheat |
| 144 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 4 | 5 | 5 | 5 | 5 | 0 | 0 | 0 | 0 | 0 |
| 151 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 153 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 165 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 4 | 4 | 3 | 0 | 0 | 0 | 0 | 0 |
| 170 | 50 | 5 | 5 | 5 | 5 | 5 | 0 | 0 | 0 | 0 | 0 |
| | 25 | 5 | 4 | 5 | 4 | 4 | 0 | 0 | 0 | 0 | 0 |
| | 12.5 | 4 | 4 | 4 | 4 | 4 | 0 | 0 | 0 | 0 | 0 |
| 176 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 178 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 182 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 4 | 0 | 0 | 0 | 0 | 0 |
| 184 | 50 | 5 | 5 | 5 | 5 | 5 | 0 | 0 | 0 | 0 | 0 |
| | 25 | 5 | 5 | 5 | 5 | 4 | 0 | 0 | 0 | 0 | 0 |
| | 12.5 | 5 | 5 | 5 | 5 | 4 | 0 | 0 | 0 | 0 | 0 |

Table 6 (Continued)

| Test compound No. | Rate of application of compound (g/10 ares) | Herbicidal effect against upland weeds | | | | | Phytotoxicity to crop plants | | | | |
|---|---|---|---|---|---|---|---|---|---|---|---|
| | | Large crabgrass | Green foxtail | Common lambusquaters | Livid amaranth | Smartweed | Soybean | Corn | Beet | Rapeseed | Wheat |
| 194 | 50 | 5 | 5 | 5 | 5 | 5 | 0 | 0 | 0 | 0 | 0 |
| | 25 | 5 | 5 | 5 | 5 | 4 | 0 | 0 | 0 | 0 | 0 |
| | 12.5 | 4 | 5 | 4 | 5 | 4 | 0 | 0 | 0 | 0 | 0 |
| 202 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 4 | 5 | 5 | 0 | 0 | 0 | 0 | 0 |
| 207 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 5 | 4 | 5 | 0 | 0 | 0 | 0 | 0 |
| 220 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 4 | 5 | 4 | 0 | 0 | 0 | 0 | 0 |
| 224 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 228 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 4 | 5 | 5 | 0 | 0 | 0 | 0 | 0 |
| 232 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 4 | 5 | 5 | 0 | 0 | 0 | 0 | 0 |
| 234 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 5 | 5 | 5 | 0 | 0 | 0 | 0 | 0 |
| 239 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 4 | 3 | 4 | 5 | 5 | 0 | 0 | 0 | 0 | 0 |

Table 6 (Continued)

| Test compound No. | Rate of application of compound (g/10 ares) | Herbicidal effect against upland weeds | | | | | Phytotoxicity to crop plants | | | | |
|---|---|---|---|---|---|---|---|---|---|---|---|
| | | Large crabgrass | Green foxtail | Common lambusquaters | Livid amaranth | Smartweed | Soybean | Corn | Beet | Rapeseed | Wheat |
| 246 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 4 | 5 | 4 | 0 | 0 | 0 | 0 | 0 |
| 248 | 50 | 5 | 5 | 5 | 5 | 5 | 0 | 0 | 0 | 0 | 0 |
| | 25 | 4 | 5 | 4 | 5 | 5 | 0 | 0 | 0 | 0 | 0 |
| | 12.5 | 3 | 4 | 4 | 5 | 5 | 0 | 0 | 0 | 0 | 0 |
| 253 | 50 | 5 | 5 | 5 | 5 | 5 | 0 | 0 | 0 | 0 | 0 |
| | 25 | 5 | 4 | 5 | 5 | 5 | 0 | 0 | 0 | 0 | 0 |
| | 12.5 | 4 | 4 | 5 | 4 | 5 | 0 | 0 | 0 | 0 | 0 |
| 254 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 4 | 4 | 5 | 0 | 0 | 0 | 0 | 0 |
| 260 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 4 | 4 | 4 | 5 | 4 | 0 | 0 | 0 | 0 | 0 |
| 261 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 4 | 5 | 4 | 0 | 0 | 0 | 0 | 0 |
| 263 | 50 | 5 | 5 | 5 | 5 | 5 | 0 | 0 | 0 | 0 | 0 |
| | 25 | 4 | 4 | 4 | 5 | 4 | 0 | 0 | 0 | 0 | 0 |
| | 12.5 | 3 | 3 | 3 | 5 | 4 | 0 | 0 | 0 | 0 | 0 |
| 264 | 50 | 5 | 5 | 5 | 5 | 5 | 0 | 0 | 0 | 0 | 0 |
| | 25 | 5 | 4 | 5 | 5 | 5 | 0 | 0 | 0 | 0 | 0 |
| | 12.5 | 4 | 3 | 4 | 4 | 4 | 0 | 0 | 0 | 0 | 0 |
| 266 | 50 | 5 | 5 | 5 | 5 | 5 | 0 | 0 | 0 | 0 | 0 |
| | 25 | 5 | 5 | 4 | 5 | 5 | 0 | 0 | 0 | 0 | 0 |
| | 12.5 | 4 | 4 | 3 | 4 | 4 | 0 | 0 | 0 | 0 | 0 |

Table 6 (Continued)

| Test compound No. | Rate of application of compound (g/10 ares) | Herbicidal effect against upland weeds | | | | | Phytotoxicity to crop plants | | | | |
|---|---|---|---|---|---|---|---|---|---|---|---|
| | | Large crabgrass | Green foxtail | Common lambusquaters | Livid amaranth | Smartweed | Soybean | Corn | Beet | Rapeseed | Wheat |
| 271 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 4 | 0 | 0 | 0 | 0 | 0 |
| 277 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 5 | 5 | 5 | 0 | 0 | 0 | 0 | 0 |
| 284 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 4 | 4 | 5 | 5 | 5 | 0 | 0 | 0 | 0 | 0 |
| 291 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 294 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 4 | 4 | 4 | 5 | 4 | 0 | 0 | 0 | 0 | 0 |
| 297 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 4 | 5 | 4 | 0 | 0 | 0 | 0 | 0 |
| 301 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 303 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 305 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 4 | 5 | 5 | 5 | 4 | 0 | 0 | 0 | 0 | 0 |

Table 6

| Test compound No. | Rate of application of compound (g/10 ares) | Herbicidal effect against upland weeds | | | | | Phytotoxicity to crop plants | | | | |
|---|---|---|---|---|---|---|---|---|---|---|---|
| | | Large crabgrass | Green foxtail | Common lambusquaters | Livid amaranth | Smartweed | Soybean | Corn | Beet | Rapeseed | Wheat |
| 309 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 4 | 5 | 4 | 0 | 0 | 0 | 0 | 0 |
| 312 | 50 | 5 | 5 | 5 | 5 | 5 | 0 | 0 | 0 | 0 | 0 |
| | 25 | 5 | 4 | 5 | 5 | 4 | 0 | 0 | 0 | 0 | 0 |
| | 12.5 | 4 | 3 | 4 | 4 | 3 | 0 | 0 | 0 | 0 | 0 |
| 315 | 50 | 5 | 5 | 5 | 5 | 5 | 0 | 0 | 0 | 0 | 0 |
| | 25 | 5 | 5 | 4 | 5 | 4 | 0 | 0 | 0 | 0 | 0 |
| | 12.5 | 4 | 4 | 3 | 4 | 3 | 0 | 0 | 0 | 0 | 0 |
| 324 | 50 | 5 | 5 | 5 | 5 | 5 | 0 | 0 | 0 | 0 | 0 |
| | 25 | 5 | 4 | 5 | 5 | 4 | 0 | 0 | 0 | 0 | 0 |
| | 12.5 | 4 | 3 | 4 | 4 | 3 | 0 | 0 | 0 | 0 | 0 |
| 329 | 50 | 5 | 5 | 5 | 5 | 5 | 0 | 0 | 0 | 0 | 0 |
| | 25 | 4 | 4 | 4 | 5 | 4 | 0 | 0 | 0 | 0 | 0 |
| | 12.5 | 3 | 3 | 3 | 5 | 3 | 0 | 0 | 0 | 0 | 0 |
| 332 | 50 | 5 | 5 | 5 | 5 | 5 | 0 | 0 | 0 | 0 | 0 |
| | 25 | 4 | 5 | 4 | 5 | 4 | 0 | 0 | 0 | 0 | 0 |
| | 12.5 | 3 | 4 | 3 | 5 | 3 | 0 | 0 | 0 | 0 | 0 |
| 335 | 50 | 5 | 5 | 5 | 5 | 5 | 0 | 0 | 0 | 0 | 0 |
| | 25 | 4 | 4 | 4 | 5 | 5 | 0 | 0 | 0 | 0 | 0 |
| | 12.5 | 3 | 3 | 3 | 5 | 4 | 0 | 0 | 0 | 0 | 0 |
| 340 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 4 | 5 | 4 | 0 | 0 | 0 | 0 | 0 |
| 344 | 50 | 5 | 5 | 5 | 5 | 5 | 0 | 0 | 0 | 0 | 0 |
| | 25 | 5 | 5 | 4 | 5 | 4 | 0 | 0 | 0 | 0 | 0 |
| | 12.5 | 4 | 4 | 3 | 5 | 3 | 0 | 0 | 0 | 0 | 0 |

Table 6 (Continued)

| Test compound No. | Rate of application of compound (g/10 ares) | Herbicidal effect against upland weeds | | | | | Phytotoxicity to crop plants | | | | |
|---|---|---|---|---|---|---|---|---|---|---|---|
| | | Large crabgrass | Green foxtail | Common lambusquaters | Livid amaranth | Smartweed | Soybean | Corn | Beet | Rapeseed | Wheat |
| 353 | 50 | 5 | 5 | 5 | 5 | 5 | 0 | 0 | 0 | 0 | 0 |
| | 25 | 5 | 5 | 4 | 5 | 4 | 0 | 0 | 0 | 0 | 0 |
| | 12.5 | 4 | 4 | 3 | 4 | 3 | 0 | 0 | 0 | 0 | 0 |
| 366 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 5 | 4 | 5 | 0 | 0 | 0 | 0 | 0 |
| 367 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 4 | 5 | 5 | 4 | 4 | 0 | 0 | 0 | 0 | 0 |
| 370 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 4 | 4 | 4 | 0 | 0 | 0 | 0 | 0 |
| 373 | 50 | 5 | 5 | 5 | 5 | 5 | 0 | 0 | 0 | 0 | 0 |
| | 25 | 5 | 5 | 4 | 5 | 5 | 0 | 0 | 0 | 0 | 0 |
| | 12.5 | 4 | 4 | 3 | 5 | 4 | 0 | 0 | 0 | 0 | 0 |
| 381 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 4 | 5 | 4 | 0 | 0 | 0 | 0 | 0 |
| 383 | 50 | 5 | 5 | 5 | 5 | 5 | 0 | 0 | 0 | 0 | 0 |
| | 25 | 5 | 4 | 5 | 5 | 5 | 0 | 0 | 0 | 0 | 0 |
| | 12.5 | 5 | 4 | 4 | 5 | 4 | 0 | 0 | 0 | 0 | 0 |
| 390 | 50 | 5 | 5 | 5 | 5 | 5 | 0 | 0 | 0 | 0 | 0 |
| | 25 | 4 | 4 | 5 | 5 | 4 | 0 | 0 | 0 | 0 | 0 |
| | 12.5 | 3 | 3 | 4 | 5 | 3 | 0 | 0 | 0 | 0 | 0 |
| 393 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 4 | 4 | 5 | 5 | 0 | 0 | 0 | 0 | 0 |

Table 6 (Continued)

| Test compound No. | Rate of application of compound (g/10 ares) | Herbicidal effect against upland weeds | | | | | Phytotoxicity to crop plants | | | | |
|---|---|---|---|---|---|---|---|---|---|---|---|
| | | Large crabgrass | Green foxtail | Common lambsquaters | Livid amaranth | Smartweed | Soybean | Corn | Beet | Rapeseed | Wheat |
| 394 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 5 | 5 | 5 | 0 | 0 | 0 | 0 | 0 |
| 402 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 4 | 5 | 5 | 0 | 0 | 0 | 0 | 0 |
| 405 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 5 | 5 | 4 | 5 | 4 | 0 | 0 | 0 | 0 | 0 |
| 409 | 50 | 5 | 5 | 5 | 5 | 5 | 0 | 0 | 0 | 0 | 0 |
| | 25 | 5 | 5 | 5 | 5 | 5 | 0 | 0 | 0 | 0 | 0 |
| | 12.5 | 4 | 4 | 4 | 5 | 3 | 0 | 0 | 0 | 0 | 0 |
| Comparative agent A | 50 | 1 | 1 | 0 | 2 | 0 | 2 | 1 | 0 | 2 | 1 |
| | 25 | 0 | 0 | 0 | 1 | 0 | 1 | 1 | 0 | 1 | 0 |
| | 12.5 | 0 | 0 | 0 | 0 | 0 | 0 | 0 | 0 | 0 | 0 |
| Comparative agent B | 50 | 1 | 1 | 0 | 1 | 0 | 1 | 1 | 0 | 2 | 2 |
| | 25 | 0 | 0 | 0 | 0 | 0 | 0 | 0 | 0 | 2 | 0 |
| | 12.5 | 0 | 0 | 0 | 0 | 0 | 0 | 0 | 0 | 0 | 0 |
| Comparative agent C | 50 | 1 | 0 | 0 | 1 | 0 | 1 | 0 | 0 | 1 | 0 |
| | 25 | 0 | 0 | 0 | 0 | 0 | 0 | 0 | 0 | 0 | 0 |
| | 12.5 | 0 | 0 | 0 | 0 | 0 | 0 | 0 | 0 | 0 | 0 |
| Comparative agent D | 50 | 0 | 0 | 0 | 1 | 0 | 0 | 0 | 0 | 1 | 0 |
| | 25 | 0 | 0 | 0 | 0 | 0 | 0 | 0 | 0 | 0 | 0 |
| | 12.5 | 0 | 0 | 0 | 0 | 0 | 0 | 0 | 0 | 0 | 0 |
| Comparative agent F | 50 | 4 | 3 | 3 | 3 | 3 | 2 | 0 | 0 | 2 | 1 |
| | 25 | 2 | 2 | 2 | 3 | 3 | 1 | 0 | 0 | 1 | 0 |
| | 12.5 | 1 | 1 | 1 | 1 | 2 | 0 | 0 | 0 | 0 | 0 |

Note: Comparative agents A to D are the same as those shown in Table 3, and Comparative agent F is the same as that shown in Table 5.

## INDUSTRIAL UTILITY OF THE INVENTION

As described hereinbefore, the new aralkyloxyamine derivatives of the above-mentioned general formula (I) having excellent herbicidal activities can be provided according to this invention, and these aralkyloxyamine derivatives are useful as such herbicide which can control or combat the weeds growing in the paddy field of rice and also the weeds growing in the fields of upland crop plants.

**Claims**

**1.** An aralkyloxyamine derivative represented by the general formula:

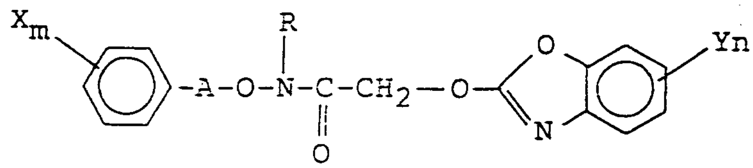

(I)

wherein X means a hydrogen atom, a halogen atom, a lower alkyl group, a lower alkoxy group, a lower haloalkyl group, a lower haloalkoxy group, a lower alkylthio group, a lower alkylsulfinyl group, a lower alkylsulfonyl group, a lower alkylcarbonyl group, a nitro group or cyano group or a lower alkoxycarbonyl group, or X means a phenoxy group which may be substituted by a halogen atom, a lower alkyl group, a lower alkoxy group or a lower haloalkyl group, m stands for 1, 2 or 3, and when a plurality of the group X are present, these groups X may be the same or different from each other, and Y denotes a hydrogen atom, a halogen atom, a lower alkyl group, a lower alkoxy group, a lower haloalkyl group, a lower haloalkoxy group, a lower alkylthio group, a lower alkylcarbonyl group, a nitro group or cyano group, n stands for 1 or 2, and when a plurality of the group Y are present, these groups Y may be the same or different from each other, and A means an alkylene group, and R represents a hydrogen atom, a lower alkyl group, a lower alkenyl group, a lower alkynyl group, a cycloalkyl group, a lower alkoxy-lower alkyl group, a lower alkylthio-lower alkyl group, a lower alkoxycarbonyl-lower alkyl group, a cyano-lower alkyl group, a lower alkylcarbonyl-lower alkyl group or a phenyl-lower alkyl group.

**2.** An aralkyloxyamine derivative as claimed in Claim 1, wherein the compound of the general formula (I) is benzoxazol-2-yloxyacetic acid N-isobutyl-N-benzyloxyamide; 6-methylbenzoxazol-2-yloxyacetic acid N-isobutyl-N-benzyloxyamide; benzoxazol-2-yloxyacetic acid N-isopropyl-N-benzyloxyamide; benzoxazol-2-yloxyacetic acid N-isopropyl-N-o-methylbenzyloxyamide; 5-methylbenzoxazol-2-yloxyacetic acid N-isopropyl-N-benzyloxyamine; 6-methylbenzoxazol-2-yloxyacetic acid N-isopropyl-N-benzyloxyamide; or benzoxazol-2-yloxyacetic acid N-isopropyl-N-m-methylbenzyloxyamide.

**3.** A herbicidal composition, characterized in that said composition comprises as an active ingredient an aralkyloxyamine derivative represented by the general formula:

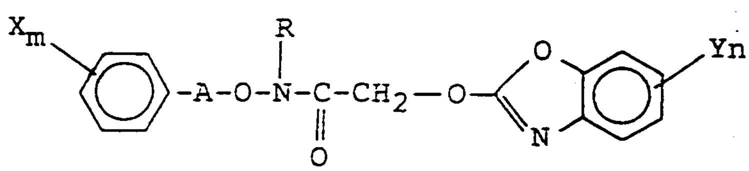

(I)

wherein X means a hydrogen atom, a halogen atom, a lower alkyl group, a lower alkoxy group, a lower haloalkyl group, a lower haloalkoxy group, a lower alkylthio group, a lower alkylsulfinyl group, a lower alkylsulfonyl group, a lower alkylcarbonyl group, a nitro group or cyano group or a lower alkoxycarbonyl group, or X means a phenoxy group which may be substituted by a halogen atom, a lower alkyl group, a lower alkoxy group or a lower haloalkyl group, m stands for 1, 2 or 3, and when a plurality of the group X are present, these groups X may be the same or different from each other, and Y denotes a hydrogen atom, a halogen atom, a lower alkyl group, a lower alkoxy group, a lower haloalkyl group, a lower haloalkoxy group, a lower alkylthio group, a lower alkylcarbonyl group, a nitro group or cyano group, n stands for 1 or 2, and when a plurality of the group Y are present, these groups Y may be the same or different from each other, and A means an alkylene group, and R represents a hydrogen atom, a lower alkyl group, a lower alkenyl group, a lower alkynyl group, a cycloalkyl group, a lower alkoxy-lower alkyl group, a lower alkylthio-lower alkyl group, a lower alkoxycarbonyl-lower alkyl group, a

cyano-lower alkyl group, a lower alkylcarbonyl-lower alkyl group or a phenyl-lower alkyl group, and a carrier as mixed with the active ingredient.

# INTERNATIONAL SEARCH REPORT

International Application No PCT/JP90/01403

**I. CLASSIFICATION OF SUBJECT MATTER** (if several classification symbols apply, indicate all) [6]

According to International Patent Classification (IPC) or to both National Classification and IPC

Int. $Cl^5$ C07D263/58, A01N43/76

**II. FIELDS SEARCHED**

Minimum Documentation Searched [7]

| Classification System | Classification Symbols |
|---|---|
| IPC | C07D263/58, A01N43/76 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 56-86176 (Bayer AG), July 13, 1981 (13. 07. 81) & DE, A, 2946524 & US, A, 4408055 | 1-3 |
| A | JP, A, 57-4903 (Bayer AG), January 11, 1982 (11. 01. 82) & DE, A, 3018075 & EP, A, 39811 | 1-3 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 17, 1991 (17. 01. 91) | January 28, 1991 (28. 01. 91) |
| **International Searching Authority** | **Signature of Authorized Officer** |
| Japanese Patent Office | |